# EUROPEAN PATENT APPLICATION

(11) **EP 2 166 111 A1**
(43) Date of publication of application: **24.03.2010**
(21) Application number: 08764890.3
(22) Date of filing: 30.05.2008
(51) Int. Cl.: C12Q 1/68, A61K 38/00, A61P 35/00, C12N 15/09, C12Q 1/02, C12Q 1/04, G01N 33/15, G01N 33/50, G01N 33/53

(54) **PROGNOSTIC FACTOR IN SARCOMA, AND METASTASIS INHIBITOR**

(30) Priority: 31.05.2007 JP 2007145827
(71) Applicant: Kyoto University, Sakyo-ku Kyoto-shi Kyoto 606-8501 (JP)
(72) Inventor: TOGUCHIDA, Junya, Kyoto-shi Kyoto 606-8507 (JP); NAGAYAMA, Satoshi, Kyoto-shi Kyoto 606-8507 (JP); FURU, Moritoshi, Kyoto-shi Kyoto 606-8507 (JP)
(74) Representative: Duckworth, Timothy John
(86) International application number: PCT/JP2008/059972
(87) International publication number: WO 2008/149782

(57) **Abstract**

The present invention relates to uses of sarcopodin as a gene associated with distant metastasis or prognosis for survival in tumors. Specifically, the present invention provides a method of determining the risk of distant metastasis or prognosis for survival in a tumor, comprising measuring the expression level of sarcopodin in a tumor tissue. Furthermore, the present invention provides an agent for inhibiting metastasis containing a polynucleotide complementary to an mRNA that encodes sarcopodin or an expression vector capable of expressing the polynucleotide.

SEQUENCE LISTING

<110> Kyoto University

<120> A factor for determining tumor prognosis and a drug for
      inhibiting metastasis

<130> 091256

<150> JP2007-145827
<151> 2007-05-31

<160> 17

<170> PatentIn version 3.2

<210> 1
<211> 4153
<212> DNA
<213> Homo sapiens


<220>
<221> CDS
<222> (75)..(2381)

<400> 1
gcgcgccggc cgctaccagc cgcgccggag cccctgcgcc ctgcggcccg ctccccgggg      60

accgggccgg cgcc atg gac cga ggc cag gtg ctg gag cag ctg ctc cca      110
                Met Asp Arg Gly Gln Val Leu Glu Gln Leu Leu Pro
                1               5                   10

gag ctc acc ggg ctg ctc agc ctc ctg gac cac gag tac ctc agc gat      158
Glu Leu Thr Gly Leu Leu Ser Leu Leu Asp His Glu Tyr Leu Ser Asp
        15                  20                  25

acc acc ctg gaa aag aag atg gcc gtg gcc tcc atc ctg cag agc ctg      206
Thr Thr Leu Glu Lys Lys Met Ala Val Ala Ser Ile Leu Gln Ser Leu
    30                  35                  40

cag ccc ctt cca gca aag gag gtc tcc tac ctg tat gtg aac aca gca      254
Gln Pro Leu Pro Ala Lys Glu Val Ser Tyr Leu Tyr Val Asn Thr Ala
45                  50                  55                  60

gac ctc cac tcg ggg ccc agc ttc gtg gaa tcc ctc ttt gaa gaa ttt      302
Asp Leu His Ser Gly Pro Ser Phe Val Glu Ser Leu Phe Glu Glu Phe
                65                  70                  75

gac tgt gac ctg agt gac ctt cgg gac atg cca gag gat gat ggg gag      350
Asp Cys Asp Leu Ser Asp Leu Arg Asp Met Pro Glu Asp Asp Gly Glu
            80                  85                  90

ccc agc aaa gga gcc agc cct gag cta gcc aag agc cca cgc ctg aga      398
Pro Ser Lys Gly Ala Ser Pro Glu Leu Ala Lys Ser Pro Arg Leu Arg
        95                  100                 105

aac gcg gcc gac ctg cct cca ccg ctc ccc aac aag cct ccc cct gag      446
Asn Ala Ala Asp Leu Pro Pro Pro Leu Pro Asn Lys Pro Pro Pro Glu
    110                 115                 120

gac tac tat gaa gag gcc ctt cct ctg gga ccc ggc aag tcg cct gag      494
Asp Tyr Tyr Glu Glu Ala Leu Pro Leu Gly Pro Gly Lys Ser Pro Glu
125                 130                 135                 140

tac atc agc tcc cac aat ggc tgc agc ccc tca cac tcg att gtg gat      542
Tyr Ile Ser Ser His Asn Gly Cys Ser Pro Ser His Ser Ile Val Asp
                145                 150                 155

ggc tac tat gag gac gca gac agc agc tac cct gca acc agg gtg aac      590
Gly Tyr Tyr Glu Asp Ala Asp Ser Ser Tyr Pro Ala Thr Arg Val Asn
            160                 165                 170

ggc gag ctt aag agc tcc tat aat gac tct gac gca atg agc agc tcc      638
Gly Glu Leu Lys Ser Ser Tyr Asn Asp Ser Asp Ala Met Ser Ser Ser

        175                 180                 185

tat gag tcc tac gat gaa gag gag gag gaa ggg aag agc ccg cag ccc 686
Tyr Glu Ser Tyr Asp Glu Glu Glu Glu Glu Gly Lys Ser Pro Gln Pro
    190                 195                 200

cga cac cag tgg ccc tca gag gag gcc tcc atg cac ctg gtg agg gaa 734
Arg His Gln Trp Pro Ser Glu Glu Ala Ser Met His Leu Val Arg Glu
205                 210                 215                 220

tgc agg ata tgt gcc ttc ctg ctg cgg aaa aag cgt ttc ggg cag tgg 782
Cys Arg Ile Cys Ala Phe Leu Leu Arg Lys Lys Arg Phe Gly Gln Trp
                225                 230                 235

gcc aag cag ctg acg gtc atc agg gag gac cag ctc ctg tgt tac aaa 830
Ala Lys Gln Leu Thr Val Ile Arg Glu Asp Gln Leu Leu Cys Tyr Lys
            240                 245                 250

agc tcc aag gat cgg cag cca cat ctg agg ttg gca ctg gat acc tgc 878
Ser Ser Lys Asp Arg Gln Pro His Leu Arg Leu Ala Leu Asp Thr Cys
        255                 260                 265

agc atc atc tac gtg ccc aag gac agc cgg cac aag agg cac gag ctg 926
Ser Ile Ile Tyr Val Pro Lys Asp Ser Arg His Lys Arg His Glu Leu
    270                 275                 280

cgt ttc acc cag ggg gct acc gag gtc ttg gtg ctg gca ctg cag agc 974
Arg Phe Thr Gln Gly Ala Thr Glu Val Leu Val Leu Ala Leu Gln Ser
285                 290                 295                 300

cga gag cag gcc gag gag tgg ctg aag gtc atc cga gaa gtg agc aag 1022
Arg Glu Gln Ala Glu Glu Trp Leu Lys Val Ile Arg Glu Val Ser Lys
                305                 310                 315

cca gtt ggg gga gct gag gga gtg gag gtc ccc aga tcc cca gtc ctc 1070
Pro Val Gly Gly Ala Glu Gly Val Glu Val Pro Arg Ser Pro Val Leu
            320                 325                 330

ctg tgc aag ttg gac ctg gac aag agg ctg tcc caa gag aag cag acc 1118
Leu Cys Lys Leu Asp Leu Asp Lys Arg Leu Ser Gln Glu Lys Gln Thr
        335                 340                 345

tca gat tct gac agc gtg ggt gtg ggt gac aac tgt tct acc ctt ggc 1166
Ser Asp Ser Asp Ser Val Gly Val Gly Asp Asn Cys Ser Thr Leu Gly
    350                 355                 360

cgc cgg gag acc tgt gat cac ggc aaa ggg aag aag agc agc ctg gca 1214
Arg Arg Glu Thr Cys Asp His Gly Lys Gly Lys Lys Ser Ser Leu Ala
365                 370                 375                 380

gaa ctg aag ggc tca atg agc agg gct gcg ggc cgc aag atc acc cgt 1262
Glu Leu Lys Gly Ser Met Ser Arg Ala Ala Gly Arg Lys Ile Thr Arg
                385                 390                 395

atc att ggc ttc tcc aag aag aag aca ctg gcc gat gac ctg cag acg 1310
Ile Ile Gly Phe Ser Lys Lys Lys Thr Leu Ala Asp Asp Leu Gln Thr
            400                 405                 410

tcc tcc acc gag gag gag gtt ccc tgc tgt ggc tac ctg aac gtg ctg 1358
Ser Ser Thr Glu Glu Glu Val Pro Cys Cys Gly Tyr Leu Asn Val Leu
        415                 420                 425

gtg aac cag ggc tgg aag gaa cgc tgg tgc cgc ctg aag tgc aac act 1406
Val Asn Gln Gly Trp Lys Glu Arg Trp Cys Arg Leu Lys Cys Asn Thr
    430                 435                 440

ctg tat ttc cac aag gat cac atg gac ctg cga acc cat gtg aac gcc 1454
Leu Tyr Phe His Lys Asp His Met Asp Leu Arg Thr His Val Asn Ala
445                 450                 455                 460

atc gcc ctg caa ggc tgt gag gtg gcc ccg ggc ttt ggg ccc cga cac 1502
Ile Ala Leu Gln Gly Cys Glu Val Ala Pro Gly Phe Gly Pro Arg His
                465                 470                 475

cca ttt gcc ttc agg atc ctg cgc aac cgg cag gag gtg gcc atc ttg 1550

Pro Phe Ala Phe Arg Ile Leu Arg Asn Arg Gln Glu Val Ala Ile Leu
            480                 485                 490

gag gca agc tgt tca gag gac atg ggt cgc tgg ctc ggg ctg ctg ctg 1598
Glu Ala Ser Cys Ser Glu Asp Met Gly Arg Trp Leu Gly Leu Leu Leu
        495                 500                 505

gtg gag atg ggc tcc aga gtc act ccg gag gcg ctg cac tat gac tac 1646
Val Glu Met Gly Ser Arg Val Thr Pro Glu Ala Leu His Tyr Asp Tyr
    510                 515                 520

gtg gat gtg gag acc tta acc agc atc gtc agt gct ggg cgc aac tcc 1694
Val Asp Val Glu Thr Leu Thr Ser Ile Val Ser Ala Gly Arg Asn Ser
525                 530                 535                 540

ttc cta tat gca aga tcc tgc cag aat cag tgg cct gag ccc cga gtc 1742
Phe Leu Tyr Ala Arg Ser Cys Gln Asn Gln Trp Pro Glu Pro Arg Val
                545                 550                 555

tat gat gat gtt cct tat gaa aag atg cag gac gag gag ccc gag cgc 1790
Tyr Asp Asp Val Pro Tyr Glu Lys Met Gln Asp Glu Glu Pro Glu Arg
            560                 565                 570

ccc aca ggg gcc cag gtg aag cgt cac gcc tcc tcc tgc agt gag aag 1838
Pro Thr Gly Ala Gln Val Lys Arg His Ala Ser Ser Cys Ser Glu Lys
        575                 580                 585

tcc cat cgt gtg gac ccg cag gtc aaa gtc aaa cgc cac gcc tcc agt 1886
Ser His Arg Val Asp Pro Gln Val Lys Val Lys Arg His Ala Ser Ser
    590                 595                 600

gcc aat caa tac aag tat ggc aag aac cga gcc gag gag gat gcc cgg 1934
Ala Asn Gln Tyr Lys Tyr Gly Lys Asn Arg Ala Glu Glu Asp Ala Arg
605                 610                 615                 620

agg tac ttg gta gaa aaa gag aag ctg gag aaa gag aaa gag acg att 1982
Arg Tyr Leu Val Glu Lys Glu Lys Leu Glu Lys Glu Lys Glu Thr Ile
                625                 630                 635

cgg aca gag ctg ata gca ctg aga cag gag aag agg gaa ctg aag gaa 2030
Arg Thr Glu Leu Ile Ala Leu Arg Gln Glu Lys Arg Glu Leu Lys Glu
            640                 645                 650

gcc att cgg agc agc cca gga gca aaa tta aag gct ctg gaa gaa gcc 2078
Ala Ile Arg Ser Ser Pro Gly Ala Lys Leu Lys Ala Leu Glu Glu Ala
        655                 660                 665

gtg gcc acc ctg gaa gct cag tgt cgg gca aag gag gag cgc cgg att 2126
Val Ala Thr Leu Glu Ala Gln Cys Arg Ala Lys Glu Glu Arg Arg Ile
    670                 675                 680

gac ctg gag ctg aag ctg gtg gct gtg aag gag cgc ttg cag cag tcc 2174
Asp Leu Glu Leu Lys Leu Val Ala Val Lys Glu Arg Leu Gln Gln Ser
685                 690                 695                 700

ctg gca gga ggg cca gcc ctg ggg ctc tcc gtg agc agc aag ccc aag 2222
Leu Ala Gly Gly Pro Ala Leu Gly Leu Ser Val Ser Ser Lys Pro Lys
                705                 710                 715

agt ggg gaa act gca aat aaa ccc cag aac agc gtt cca gag caa cct 2270
Ser Gly Glu Thr Ala Asn Lys Pro Gln Asn Ser Val Pro Glu Gln Pro
            720                 725                 730

ctc cct gtc aac tgt gtt tct gag ctg agg aag agg agc cca tcc atc 2318
Leu Pro Val Asn Cys Val Ser Glu Leu Arg Lys Arg Ser Pro Ser Ile
        735                 740                 745

gta gcc tcc aac caa gga agg gtg cta cag aaa gcc aag gaa tgg gaa 2366
Val Ala Ser Asn Gln Gly Arg Val Leu Gln Lys Ala Lys Glu Trp Glu
    750                 755                 760

atg aag aag acc tag gaagaggatg aggatttcat tccaaaggaa ataccagctt 2421
Met Lys Lys Thr
765

gtccacctga ggaagaaatg cttttttcac aaggagacac caagagaaga ctaggaagta 2481
gccctcgttc tccagggcac ccaaaatacc agcctttatt gtctgcatga ttttagggga 2541
tatggggagg gaacaagtag aagggaagag ggaaatggag agcatcctta tgactttaca 2601
aagggtggaa atgaggatgg agggatacag aagtctgcac agctgtaaag gttttataga 2661
tgtctttgcc ttcccttctg aggaagggaa gaagtaatga aagcacatgt gaataacccc 2721
ttccatccca ttcacagcat cgcactccca gtccttaagg caaagggagg cagtgctgaa 2781
gcattggtgg tgcagtgtaa agagacaaga cctgatcatc tgatcacact tgtgccaact 2841
tgattcatat tgggcattac taacaacccc tgggcaaggt aaataggttg aacaatcaat 2901
aacattatcc ctgcctgcat acatgtgaac aaaagctata gaggacatgc aaattctaca 2961
gtcattcctc atatgcttta gacagagtgc agctactgga atcttccaga tttcagtgtt 3021
ttaaaatcag agctctgaat acacaaaagg aaagagaaat ggagcagctg acatatttta 3081
agctcacagt gatactcagt gacaggagca cagagctcta atgtccacag gatgttgtag 3141
ggtagggtct ctcagtaaat caagtccctt acctatgttc tgacactgag gctcttggag 3201
ctatgggtta gaaatccagg aggcaatatg tctttattct aatgaagtcc tcatcttgca 3261
ctcagaggcc cactagtttg cccttctata tattaagtaa aaccaagaga aattaagagg 3321
atgatggctt ctttctgtca catgggtgct aaacctttta caaacacttg atacttctat 3381
agagggacca aggacctcaa gtcctgggca gtctccttac tgtgctactc actactgtct 3441
caacactgta gtgagtctgt ctttaacaca tttccttcca ataaagataa aaccacttaa 3501
ctcttaagtg ccagtgctta gctcaagagg aaagaaggaa aatcccagaa taaccgtggc 3561
cactttgcta taggggttac atattttaac ccacctaagg ttttacagac tttagcctca 3621
ctcaaacttt atcaccctct gaggtagctg gagaccacag caggaaaaat aacctgacca 3681
gtaatcaccc actgattagt ggcagagcgt aggacttagg ggttataagc ccagatccag 3741
atagggctag gtttgagcca tgcctctaat gaccagctgg gtgaccttgg gaaagtcact 3801
tgattgctct gattcttgtt ttccttatct gtgaaatggg actaacaatt gttacttttc 3861
tcccaagatt gttgggaaga tttaataaga taatatatag taggcatctt gcatagtgtc 3921
tgagaaataa gaaatgctca gtcaatagtg gctctcatta gccaggcatg gtggtactcg 3981
cctgtagtcc cagctactca ggaggctgag gcaggaggac cgcttgagcc caggagtttg 4041
agactgtagt gcactatgat cacgcctgtg aattgccact gcactccagc ctaagcaaat 4101
agcaagaccc catctattaa aaaaaaaaag tggctattat tagctatatc ag 4153

<210> 2
<211> 768
<212> PRT
<213> Homo sapiens

<400> 2

Met Asp Arg Gly Gln Val Leu Glu Gln Leu Leu Pro Glu Leu Thr Gly
1               5                   10                  15

Leu Leu Ser Leu Leu Asp His Glu Tyr Leu Ser Asp Thr Thr Leu Glu
            20                  25                  30

Lys Lys Met Ala Val Ala Ser Ile Leu Gln Ser Leu Gln Pro Leu Pro

        35                  40                  45

Ala Lys Glu Val Ser Tyr Leu Tyr Val Asn Thr Ala Asp Leu His Ser
    50                  55                  60

Gly Pro Ser Phe Val Glu Ser Leu Phe Glu Glu Phe Asp Cys Asp Leu
65                  70                  75                  80

Ser Asp Leu Arg Asp Met Pro Glu Asp Asp Gly Glu Pro Ser Lys Gly
                85                  90                  95

Ala Ser Pro Glu Leu Ala Lys Ser Pro Arg Leu Arg Asn Ala Ala Asp
            100                 105                 110

Leu Pro Pro Pro Leu Pro Asn Lys Pro Pro Pro Glu Asp Tyr Tyr Glu
        115                 120                 125

Glu Ala Leu Pro Leu Gly Pro Gly Lys Ser Pro Glu Tyr Ile Ser Ser
    130                 135                 140

His Asn Gly Cys Ser Pro Ser His Ser Ile Val Asp Gly Tyr Tyr Glu
145                 150                 155                 160

Asp Ala Asp Ser Ser Tyr Pro Ala Thr Arg Val Asn Gly Glu Leu Lys
                165                 170                 175

Ser Ser Tyr Asn Asp Ser Asp Ala Met Ser Ser Ser Tyr Glu Ser Tyr
            180                 185                 190

Asp Glu Glu Glu Glu Glu Gly Lys Ser Pro Gln Pro Arg His Gln Trp
        195                 200                 205

Pro Ser Glu Glu Ala Ser Met His Leu Val Arg Glu Cys Arg Ile Cys
    210                 215                 220

Ala Phe Leu Leu Arg Lys Lys Arg Phe Gly Gln Trp Ala Lys Gln Leu
225                 230                 235                 240

Thr Val Ile Arg Glu Asp Gln Leu Leu Cys Tyr Lys Ser Ser Lys Asp
                245                 250                 255

Arg Gln Pro His Leu Arg Leu Ala Leu Asp Thr Cys Ser Ile Ile Tyr
            260                 265                 270

Val Pro Lys Asp Ser Arg His Lys Arg His Glu Leu Arg Phe Thr Gln
        275                 280                 285

Gly Ala Thr Glu Val Leu Val Leu Ala Leu Gln Ser Arg Glu Gln Ala
    290                 295                 300

Glu Glu Trp Leu Lys Val Ile Arg Glu Val Ser Lys Pro Val Gly Gly
305                 310                 315                 320

Ala Glu Gly Val Glu Val Pro Arg Ser Pro Val Leu Leu Cys Lys Leu
                325                 330                 335

Asp Leu Asp Lys Arg Leu Ser Gln Glu Lys Gln Thr Ser Asp Ser Asp
            340                 345                 350

Ser Val Gly Val Gly Asp Asn Cys Ser Thr Leu Gly Arg Arg Glu Thr
        355                 360                 365

Cys Asp His Gly Lys Gly Lys Lys Ser Ser Leu Ala Glu Leu Lys Gly
    370                 375                 380

Ser Met Ser Arg Ala Ala Gly Arg Lys Ile Thr Arg Ile Ile Gly Phe
385                 390                 395                 400

Ser Lys Lys Lys Thr Leu Ala Asp Asp Leu Gln Thr Ser Ser Thr Glu
                405                 410                 415

Glu Glu Val Pro Cys Cys Gly Tyr Leu Asn Val Leu Val Asn Gln Gly
            420                 425                 430

Trp Lys Glu Arg Trp Cys Arg Leu Lys Cys Asn Thr Leu Tyr Phe His
        435                 440                 445

Lys Asp His Met Asp Leu Arg Thr His Val Asn Ala Ile Ala Leu Gln
    450                 455                 460

Gly Cys Glu Val Ala Pro Gly Phe Gly Pro Arg His Pro Phe Ala Phe
465                 470                 475                 480

Arg Ile Leu Arg Asn Arg Gln Glu Val Ala Ile Leu Glu Ala Ser Cys
                485                 490                 495

Ser Glu Asp Met Gly Arg Trp Leu Gly Leu Leu Leu Val Glu Met Gly
            500                 505                 510

Ser Arg Val Thr Pro Glu Ala Leu His Tyr Asp Tyr Val Asp Val Glu
        515                 520                 525

Thr Leu Thr Ser Ile Val Ser Ala Gly Arg Asn Ser Phe Leu Tyr Ala
    530                 535                 540

Arg Ser Cys Gln Asn Gln Trp Pro Glu Pro Arg Val Tyr Asp Asp Val
545                 550                 555                 560

Pro Tyr Glu Lys Met Gln Asp Glu Glu Pro Glu Arg Pro Thr Gly Ala
                565                 570                 575

Gln Val Lys Arg His Ala Ser Ser Cys Ser Glu Lys Ser His Arg Val
            580                 585                 590

Asp Pro Gln Val Lys Val Lys Arg His Ala Ser Ser Ala Asn Gln Tyr
        595                 600                 605

Lys Tyr Gly Lys Asn Arg Ala Glu Glu Asp Ala Arg Arg Tyr Leu Val
    610                 615                 620

Glu Lys Glu Lys Leu Glu Lys Glu Lys Glu Thr Ile Arg Thr Glu Leu
625                 630                 635                 640

Ile Ala Leu Arg Gln Glu Lys Arg Glu Leu Lys Glu Ala Ile Arg Ser
                645                 650                 655

Ser Pro Gly Ala Lys Leu Lys Ala Leu Glu Glu Ala Val Ala Thr Leu
            660                 665                 670

Glu Ala Gln Cys Arg Ala Lys Glu Glu Arg Arg Ile Asp Leu Glu Leu
        675                 680                 685

Lys Leu Val Ala Val Lys Glu Arg Leu Gln Gln Ser Leu Ala Gly Gly
    690                 695                 700

Pro Ala Leu Gly Leu Ser Val Ser Ser Lys Pro Lys Ser Gly Glu Thr
705                 710                 715                 720

Ala Asn Lys Pro Gln Asn Ser Val Pro Glu Gln Pro Leu Pro Val Asn
                725                 730                 735

Cys Val Ser Glu Leu Arg Lys Arg Ser Pro Ser Ile Val Ala Ser Asn
            740                 745                 750

Gln Gly Arg Val Leu Gln Lys Ala Lys Glu Trp Glu Met Lys Lys Thr
        755                 760                 765

<210> 3
<211> 2586
<212> DNA
<213> Homo sapiens

<220>
<221> CDS
<222> (317)..(1468)

<400> 3
   ctaagtcatg gttgtctttt caatacctgg tatggtatga ggcacaggat agttgctgac 60
   aaacatttgt tagctaatta tcatgtgctg ggggagttgg agggagattg aaccaataaa 120
   gggaaatttg ggtacttctg aatgtgccct ctgatgaggg actgcctgtg tccctcctca 180
   gaggctgtcc caagagaagc agacctcaga ttctgacagc gtgggtgtgg gtgacaactg 240
   ttctaccctt ggccgccggg agacctgtga tcacggcaaa gggaagaaga gcagcctggc 300
   agaactgaag ggctca atg agc agg gct gcg ggc cgc aag atc acc cgt atc 352
   Met Ser Arg Ala Ala Gly Arg Lys Ile Thr Arg Ile
   1 5 10
   att ggc ttc tcc aag aag aag aca ctg gcc gat gac ctg cag acg tcc 400
   Ile Gly Phe Ser Lys Lys Lys Thr Leu Ala Asp Asp Leu Gln Thr Ser
   15 20 25
   tcc acc gag gag gag gtt ccc tgc tgt ggc tac ctg aac gtg ctg gtg 448
   Ser Thr Glu Glu Glu Val Pro Cys Cys Gly Tyr Leu Asn Val Leu Val
   30 35 40
   aac cag ggc tgg aag gaa cgc tgg tgc cgc ctg aag tgc aac act ctg 496
   Asn Gln Gly Trp Lys Glu Arg Trp Cys Arg Leu Lys Cys Asn Thr Leu
   45 50 55 60
   tat ttc cac aag gat cac atg gac ctg cga acc cat gtg aac gcc atc 544
   Tyr Phe His Lys Asp His Met Asp Leu Arg Thr His Val Asn Ala Ile
   65 70 75
   gcc ctg caa ggc tgt gag gtg gcc ccg ggc ttt ggg ccc cga cac cca 592
   Ala Leu Gln Gly Cys Glu Val Ala Pro Gly Phe Gly Pro Arg His Pro
   80 85 90
   ttt gcc ttc agg atc ctg cgc aac cgg cag gag gtg gcc atc ttg gag 640
   Phe Ala Phe Arg Ile Leu Arg Asn Arg Gln Glu Val Ala Ile Leu Glu
   95 100 105
   gca agc tgt tca gag gac atg ggt cgc tgg ctc ggg ctg ctg ctg gtg 688
   Ala Ser Cys Ser Glu Asp Met Gly Arg Trp Leu Gly Leu Leu Leu Val
   110 115 120
   gag atg ggc tcc aga gtc act ccg gag gcg ctg cac tat gac tac gtg 736
   Glu Met Gly Ser Arg Val Thr Pro Glu Ala Leu His Tyr Asp Tyr Val
   125 130 135 140
   gat gtg gag acc tta acc agc atc gtc agt gct ggg cgc aac tcc ttc 784
   Asp Val Glu Thr Leu Thr Ser Ile Val Ser Ala Gly Arg Asn Ser Phe
   145 150 155
   cta tat gca aga tcc tgc cag aat cag tgg cct gag ccc cga gtc tat 832
   Leu Tyr Ala Arg Ser Cys Gln Asn Gln Trp Pro Glu Pro Arg Val Tyr
   160 165 170
   gat gat gtt cct tat gaa aag atg cag gac gag gag ccc gag cgc ccc 880
   Asp Asp Val Pro Tyr Glu Lys Met Gln Asp Glu Glu Pro Glu Arg Pro
   175 180 185
   aca ggg gcc cag gtg aag cgt cac gcc tcc tcc tgc agt gag aag tcc 928
   Thr Gly Ala Gln Val Lys Arg His Ala Ser Ser Cys Ser Glu Lys Ser
   190 195 200
   cat cgt gtg gac ccg cag gtc aaa gtc aaa cgc cac gcc tcc agt gcc 976
   His Arg Val Asp Pro Gln Val Lys Val Lys Arg His Ala Ser Ser Ala
   205 210 215 220
   aat caa tac aag tat ggc aag aac cga gcc gag gag gat gcc cgg agg 1024
   Asn Gln Tyr Lys Tyr Gly Lys Asn Arg Ala Glu Glu Asp Ala Arg Arg
   225 230 235
   tac ttg gta gaa aaa gag aag ctg gag aaa gag aaa gag acg att cgg 1072
   Tyr Leu Val Glu Lys Glu Lys Leu Glu Lys Glu Lys Glu Thr Ile Arg
   240 245 250
   aca gag ctg ata gca ctg aga cag gag aag agg gaa ctg aag gaa gcc 1120
   Thr Glu Leu Ile Ala Leu Arg Gln Glu Lys Arg Glu Leu Lys Glu Ala
   255 260 265
   att cgg agc agc cca gga gca aaa tta aag gct ctg gaa gaa gcc gtg 1168
   Ile Arg Ser Ser Pro Gly Ala Lys Leu Lys Ala Leu Glu Glu Ala Val
   270 275 280
   gcc acc ctg gaa gct cag tgt cgg gca aag gag gag cgc cgg att gac 1216
   Ala Thr Leu Glu Ala Gln Cys Arg Ala Lys Glu Glu Arg Arg Ile Asp
   285 290 295 300
   ctg gag ctg aag ctg gtg gct gtg aag gag cgc ttg cag cag tcc ctg 1264
   Leu Glu Leu Lys Leu Val Ala Val Lys Glu Arg Leu Gln Gln Ser Leu
   305 310 315
   gca gga ggg cca gcc ctg ggg ctc tcc gtg agc agc aag ccc aag agt 1312
   Ala Gly Gly Pro Ala Leu Gly Leu Ser Val Ser Ser Lys Pro Lys Ser
   320 325 330
   ggg gaa act gca aat aaa ccc cag aac agc gtt cca gag caa cct ctc 1360
   Gly Glu Thr Ala Asn Lys Pro Gln Asn Ser Val Pro Glu Gln Pro Leu
   335 340 345
   cct gtc aac tgt gtt tct gag ctg agg aag agg agc cca tcc atc gta 1408
   Pro Val Asn Cys Val Ser Glu Leu Arg Lys Arg Ser Pro Ser Ile Val
   350 355 360
   gcc tcc aac caa gga agg gtg cta cag aaa gcc aag gaa tgg gaa atg 1456
   Ala Ser Asn Gln Gly Arg Val Leu Gln Lys Ala Lys Glu Trp Glu Met
   365 370 375 380
   aag aag acc tag gaagaggatg aggatttcat tccaaaggaa ataccagctt 1508
   Lys Lys Thr
   gtccacctga ggaagaaatg cttttttcac aaggagacac caagagaaga ctaggaagta 1568
   gccctcgttc tccagggcac ccaaaatacc agcctttatt gtctgcatga ttttagggga 1628
   tatggggagg gaacaagtag aagggaagag ggaaatggag agcatcctta tgactttaca 1688
   aagggtggaa atgaggatgg agggatacag aagtctgcac agctgtaaag gttttataga 1748
   tgtctttgcc ttcccttctg aggaagggaa gaagtaatga aagcacatgt gaataacccc 1808
   ttccatccca ttcacagcat cgcactccca gtccttaagg caaagggagg cagtgctgaa 1868
   gcattggtgg tgcagtgtaa agagacaaga cctgatcatc tgatcacact tgtgccaact 1928
   tgattcatat tgggcattac taacaacccc tgggcaaggt aaataggttg aacaatcaat 1988
   aacattatcc ctgcctgcat acatgtgaac aaaagctatt gaggacatgc aaattctaca 2048
   gtcattcctc atatgcttta gacagagtgc agctactgga atcttccaga tttcagtgtt 2108
   ttaaaatcag agctctgaat acacaaaagg aaagagaaat ggagcggctg acatatttta 2168
   agctcacagt gatactcagt gacaggagca cagagctcta atgtccacag gatgtcgtag 2228
   ggtagggtct ctcagtaaat caagtccctt acctatgttc tgacactgag gctcttggag 2288
   ctatgggtta gaaatccagg aggcaatatg tctttattct aatgaagtcc tcatcttgca 2348
   ctcagaggcc cactagtttg cccttctata tattaagtaa aaccaagaga aattaagagg 2408
   atgatggctt ctttctgtca catgggtgct aaacctttta caaacacttg atacttctat 2468
   agagggacca aggacctcaa gtcctgggca gtctccttac tgtgctactc actactgtct 2528
   caacactgta gtgagtctgt ctttaacaca tttccttcca ataaagataa aaccactt 2586

<210> 4
<211> 383
<212> PRT
<213> Homo sapiens

<400> 4

Met Ser Arg Ala Ala Gly Arg Lys Ile Thr Arg Ile Ile Gly Phe Ser
1               5                   10                  15

Lys Lys Lys Thr Leu Ala Asp Asp Leu Gln Thr Ser Ser Thr Glu Glu
            20                  25                  30

Glu Val Pro Cys Cys Gly Tyr Leu Asn Val Leu Val Asn Gln Gly Trp
        35                  40                  45

Lys Glu Arg Trp Cys Arg Leu Lys Cys Asn Thr Leu Tyr Phe His Lys
    50                  55                  60

Asp His Met Asp Leu Arg Thr His Val Asn Ala Ile Ala Leu Gln Gly
65                  70                  75                  80

Cys Glu Val Ala Pro Gly Phe Gly Pro Arg His Pro Phe Ala Phe Arg
                85                  90                  95

Ile Leu Arg Asn Arg Gln Glu Val Ala Ile Leu Glu Ala Ser Cys Ser
            100                 105                 110

Glu Asp Met Gly Arg Trp Leu Gly Leu Leu Leu Val Glu Met Gly Ser
        115                 120                 125

Arg Val Thr Pro Glu Ala Leu His Tyr Asp Tyr Val Asp Val Glu Thr
    130                 135                 140

Leu Thr Ser Ile Val Ser Ala Gly Arg Asn Ser Phe Leu Tyr Ala Arg
145                 150                 155                 160

Ser Cys Gln Asn Gln Trp Pro Glu Pro Arg Val Tyr Asp Asp Val Pro
                165                 170                 175

Tyr Glu Lys Met Gln Asp Glu Glu Pro Glu Arg Pro Thr Gly Ala Gln
            180                 185                 190

Val Lys Arg His Ala Ser Ser Cys Ser Glu Lys Ser His Arg Val Asp
        195                 200                 205

Pro Gln Val Lys Val Lys Arg His Ala Ser Ser Ala Asn Gln Tyr Lys
    210                 215                 220

Tyr Gly Lys Asn Arg Ala Glu Glu Asp Ala Arg Arg Tyr Leu Val Glu
225                 230                 235                 240

Lys Glu Lys Leu Glu Lys Glu Lys Glu Thr Ile Arg Thr Glu Leu Ile
                245                 250                 255

Ala Leu Arg Gln Glu Lys Arg Glu Leu Lys Glu Ala Ile Arg Ser Ser
            260                 265                 270

Pro Gly Ala Lys Leu Lys Ala Leu Glu Glu Ala Val Ala Thr Leu Glu
        275                 280                 285

Ala Gln Cys Arg Ala Lys Glu Glu Arg Arg Ile Asp Leu Glu Leu Lys
    290                 295                 300

Leu Val Ala Val Lys Glu Arg Leu Gln Gln Ser Leu Ala Gly Gly Pro
305                 310                 315                 320

Ala Leu Gly Leu Ser Val Ser Ser Lys Pro Lys Ser Gly Glu Thr Ala
                325                 330                 335

Asn Lys Pro Gln Asn Ser Val Pro Glu Gln Pro Leu Pro Val Asn Cys
            340                 345                 350

Val Ser Glu Leu Arg Lys Arg Ser Pro Ser Ile Val Ala Ser Asn Gln
        355                 360                 365

Gly Arg Val Leu Gln Lys Ala Lys Glu Trp Glu Met Lys Lys Thr
    370                 375                 380

<210>  5
<211>  26
<212>  DNA
<213>  Artificial

<220>

<223>  primer for human sarcopodin

<400>  5
tggtggtgca gtgtaaagag acaaga                                          26


<210>  6
<211>  22
<212>  DNA
<213>  Artificial

<220>
<223>  primer for human sarcopodin

<400>  6
gtttagcacc catgtgacag aa                                              22


<210>  7
<211>  23
<212>  DNA
<213>  Artificial

<220>
<223>  primer for long form human sarcopodin

<400>  7
atcggcagcc acatctgagg ttg                                             23


<210>  8
<211>  20
<212>  DNA
<213>  Artificial

<220>
<223>  primer for long form human sarcopodin

<400>  8
ttgtcaccca cacccacgct                                                 20


<210>  9
<211>  23
<212>  DNA
<213>  Artificial

<220>
<223>  primer for short form human sarcopodin

<400>  9
gtatggtatg aggcacagga tag                                             23


<210>  10
<211>  20
<212>  DNA
<213>  Artificial

<220>
<223>  primer for short form human sarcopodin

<400>  10
gcgttcacat gggttcgcag                                                 20


<210>  11
<211>  22
<212>  DNA
<213>  Artificial

<220>
<223>  primer for human sarcopodin

<400>  11

aacaagcctc cccctgagga ct                                                22

<210>  12
<211>  20
<212>  DNA
<213>  Artificial

<220>
<223>  primer for human sarcopodin

<400>  12
gttgcagggt agctgctgtc                                                   20

<210>  13
<211>  22
<212>  DNA
<213>  Artificial

<220>
<223>  TaqMan probe for human sarcopodin

<400>  13
ggcccttcct ctgggacccg gc                                                22

<210>  14
<211>  21
<212>  RNA
<213>  Artificial

<220>
<223>  siRNA for human sarcopodin

<400>  14
cagggugaac ggcgagcuua a                                                 21

<210>  15
<211>  21
<212>  RNA
<213>  Artificial

<220>
<223>  siRNA for human sarcopodin

<400>  15
cagcaucauc uacgugccca a                                                 21

<210>  16
<211>  21
<212>  RNA
<213>  Artificial

<220>
<223>  siRNA for human sarcopodin

<400>  16
aucccucuuu gaagaauuug a                                                 21

<210>  17
<211>  21
<212>  RNA
<213>  Artificial

<220>
<223>  siRNA for human sarcopodin

<400>  17
aaaggagguc uccuaccugu a                                                 21

## Description

### Technical Field

The present invention relates to uses of a gene associated with distant metastasis of a tumor. More specifically, the present invention relates to a method of determining the risk of distant metastasis and the prognosis for survival in spindle cell sarcoma or in colorectal cancer by utilizing the gene, a medicine for inhibiting the metastasis of a sarcoma or the motility or invasive potential of sarcoma cells, a method of screening for the medicine, and the like.

### Background Art

Malignant soft tissue tumors, particularly a class of sarcomas generically referred to as spindle cell sarcoma, are pathologically highly diverse, and their classification remains unestablished definitely. Therefore, it is considerably difficult to perform a prognostic factorial analysis by pathologic diagnostic category. Regarding the determination of the malignancy, some grading systems with cell division profiles, necrotic profiles and the like as indexes have been proposed, but there is no index that enables foreseeing the onset of distant metastasis, which determines the prognosis for survival of the patient. For this reason, treatment by the same protocol must be given to all cases; otherwise unnecessary adjuvant therapy is performed even for patients with a good prognosis. Additionally, for patients with a poor prognosis, who should receive more proactive treatment, the treatment can sometimes be inadequate. Hence, there is a demand for the development of a method of determining the prognosis for survival or the risk of onset of distant metastasis in spindle cell sarcoma.

Regarding the local recurrence of spindle cell sarcoma, prevention has been increasingly possible thanks to the establishment and spread of the concept of extensive resection. However, for the onset of distant metastasis, which determines the prognosis for survival in spindle cell sarcoma, chemotherapy is mainly applied since the significance of topical therapy is low. Currently, a therapeutic method using a plurality of anticancer agents in combination is used as a standard protocol, but the response rate thereof remains up to 30%. For improvement of the prognosis, a therapeutic method aiming at suppression of metastasis is thought to be theoretically the most promising; to date, however, no factor related to the metastasis of spindle cell sarcoma has been identified, so that no therapy targeting such a metastasis-related factor has been developed.

In colorectal cancer, it is routine practice that an initial diagnosis is made by fecal occult blood reactions, endoscopic examination and the like, and a final diagnosis made by biopsy. Since a pathologic diagnosis based on biopsy sample histology requires high skills, there is a demand for the development of a diagnostic marker for facilitating the diagnosis thereof. Furthermore, there is another demand for the development of a method of determining the prognosis for survival or the risk of onset of distant metastasis in colorectal cancer patients.

Meanwhile, as genes that have been obtained as a result of a comprehensive cDNA acquirement examination, a gene under accession number AK074185 (as a protein, FLJ00258) and a gene under accession number AK094067 are registered with GenBank (Non-patent Document 1), but no report is available on the biological functions or clinical medical significance thereof.

It has been reported that a protein called AFAP-110 is a substrate for pp60src bound to the actin filament, and a molecule that regulates the integrity of the actin filament (Non-patent Documents 2 and 3). However, the involvement of AFAP-110 in cancer metastasis has not been reported.
Non-patent Document 1: Nature Genetics, vol.36(1), p.40-45, 2004
Non-patent Document 2: Mol. Cell Biol., vol.13(12), p.7892-7900, 1993
Non-patent Document 3: Oncogene, vol.16(17), p.2185-2195, 1998

### Disclosure of the Invention

### Problems to Be Solved by the Invention

It is a first object of the present invention to identify genes associated with distant metastasis of tumors such as spindle cell sarcoma or the prognosis for survival in said tumors.
It is a second object of the present invention to provide a method of determining the risk of onset of distant metastasis of tumors such as spindle cell sarcoma or the prognosis for survival in said tumors.
It is a third object of the present invention to provide a medicine capable of suppressing metastases that have a major impact on the prognosis for survival in tumors such as spindle cell sarcoma.

### Means for Solving the Problems

The present inventors performed a comprehensive gene expression analysis on spindle cell sarcoma tissue, and attempted to identify genes associated with the onset of distant metastasis and the occurrence of tumor-related deaths. As a result, the present inventors found that in tumors wherein a functionally unknown gene registered with GenBank as AK074185 (as a protein, FLJ00258) is highly expressed, the incidence of distant metastasis is high and the prognosis for survival is poor. Functional analysis revealed that the gene product is a protein that binds to actin and Src and mediates the motility and invasive potential of cells, and that by suppressing the expression thereof using an siRNA, the motility and invasive potential of sarcoma cells are suppressed. Furthermore, the inventors obtained a result showing that the shorter form, which consists of a carboxyl-terminal portion only, registered as AK094067, exists in this gene, which, by acting competitively on the longer form, possibly suppresses the motility and invasive potential of sarcoma cells.
Furthermore, analyzing the expression of this gene in colorectal mucosal tissue, it was found that in normal tissue and polyps, this gene was little expressed, but was expressed specifically in colorectal cancer cells. In colorectal cancer cells of low heterogeneity, this gene product was localized in the nucleus, whereas in tumor cells of high heterogeneity, this gene product was localized in the cytoplasm; it was found that as the malignancy of colorectal cancer cells changed, the intracellular localization of this gene product changed.
On the basis of these findings, the present invention has been developed.

Accordingly, the present invention relates to the following:
[1] A method of determining the risk of distant metastasis of a tumor, comprising measuring the expression level of any polypeptide or polynucleotide selected from among (1) to (5) below in tumor tissue, and determining the risk of distant metastasis of the tumor on the basis of the correlation between the expression level and the incidence of distant metastasis:
   (1) a polypeptide comprising the amino acid sequence depicted in SEQ ID NO:2;
   (2) a polypeptide comprising an amino acid sequence having an identity of 90% or more to the amino acid sequence depicted in SEQ ID NO:2, and possessing an activity to enhance the motility or invasive potential of a cell when expressed in the cell;
   (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (4) a polynucleotide comprising a coding region in the nucleotide sequence depicted in SEQ ID NO:1; and
   (5) a polynucleotide comprising a nucleotide sequence that encodes any polypeptide selected from among (1) to (3) above.
[2] The method described in [1], wherein the expression level is measured using any substance selected from among (i) to (iii) below:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[3] The method described in [2], wherein the substance used for the measurement is any one selected from among (ia) to (iiia) below:
   (ia) an antibody that specifically recognizes a polypeptide consisting of the amino acid sequence depicted in SEQ ID NO:2,
   a recognition epitope for the antibody being present within the region shown by amino acid numbers 1 to 385 in SEQ ID NO:2;
   (iia) a nucleic acid probe or primer capable of specifically detecting a polynucleotide consisting of the nucleotide sequence shown by nucleotide numbers 1 to 1229 in SEQ ID NO:1; and
   (iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.
[4] The method described in any one of [1] to [3], wherein the tumor is spindle cell sarcoma or colorectal cancer.
[5] A method of determining the prognosis for survival of a tumor patient, comprising measuring the expression level of any polypeptide or polynucleotide selected from among (1) to (5) below in tumor tissue, and determining the prognosis for survival of the tumor patient on the basis of the correlation between the expression level and the determination of the prognosis for survival of the tumor patient:
   (1) a polypeptide comprising the amino acid sequence depicted in SEQ ID NO:2;
   (2) a polypeptide comprising an amino acid sequence having an identity of 90% or more to the amino acid sequence depicted in SEQ ID NO:2, and possessing an activity to enhance the motility or invasive potential of a cell when expressed in the cell;
   (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (4) a polynucleotide comprising a coding region in the nucleotide sequence depicted in SEQ ID NO:1; and
   (5) a polynucleotide comprising a nucleotide sequence that encodes any polypeptide selected from among (1) to (3) above.
[6] The method described in [5], wherein the expression level is measured using any substance selected from among (i) to (iii) below:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[7] The method described in [6], wherein the substance used for the measurement is any one selected from among (ia) to (iiia) below:
   (ia) an antibody that specifically recognizes a polypeptide consisting of the amino acid sequence depicted in SEQ ID NO:2,
   a recognition epitope for the antibody being present within the region shown by amino acid numbers 1 to 385 in SEQ ID NO:2;
   (iia) a nucleic acid probe or primer capable of specifically detecting a polynucleotide consisting of the nucleotide sequence shown by nucleotide numbers 1 to 1229 in SEQ ID NO:1; and
   (iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.
[8] The method described in any one of [5] to [7], wherein the tumor is spindle cell sarcoma or colorectal cancer.
[9] An agent for determining the risk of distant metastasis of a tumor, comprising any substance selected from among (i) to (iii) below:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[10] The agent described in [9], wherein the substance is any one selected from among (ia) to (iiia) below:
   (ia) an antibody that specifically recognizes a polypeptide consisting of the amino acid sequence depicted in SEQ ID NO:2, a recognition epitope for the antibody being present within the region shown by amino acid numbers 1 to 385 in SEQ ID NO:2;
   (iia) a nucleic acid probe or primer capable of specifically detecting a polynucleotide consisting of the nucleotide sequence shown by nucleotide numbers 1 to 1229 in SEQ ID NO:1; and
   (iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.
[11] The agent described in [9] or [10], wherein the tumor is spindle cell sarcoma or colorectal cancer.
[12] An agent for determining the prognosis for survival of a tumor patient, comprising any substance selected from among (i) to (iii) below:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[13] The agent described in [12], wherein the substance is any one selected from among (ia) to (iiia) below:
   (ia) an antibody that specifically recognizes a polypeptide consisting of the amino acid sequence depicted in SEQ ID NO:2, a recognition epitope for the antibody being present within the region shown by amino acid numbers 1 to 385 in SEQ ID NO:2;
   (iia) a nucleic acid probe or primer capable of specifically detecting a polynucleotide consisting of the nucleotide sequence shown by nucleotide numbers 1 to 1229 in SEQ ID NO:1; and
   (iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.
[14] The agent described in [12] or [13], wherein the tumor is spindle cell sarcoma or colorectal cancer.
[15] A method of detecting colorectal cancer cells in colorectal mucosal tissue, comprising evaluating the expression of any polypeptide or polynucleotide selected from among (1) to (5) below in colorectal mucosal tissue, and determining whether or not colorectal cancer cells are contained in the tissue on the basis of the presence or absence of the expression:
   (1) a polypeptide comprising the amino acid sequence depicted in SEQ ID NO:2;
   (2) a polypeptide comprising an amino acid sequence having an identity of 90% or more to the amino acid sequence depicted in SEQ ID NO:2, and possessing an activity to enhance the motility or invasive potential of a cell when expressed in the cell;
   (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (4) a polynucleotide comprising a coding region in the nucleotide sequence depicted in SEQ ID NO:1; and
   (5) a polynucleotide comprising a nucleotide sequence that encodes any polypeptide selected from among (1) to (3) above.
[16] An agent for detecting colorectal cancer cells in colorectal mucosal tissue, comprising any substance selected from among (i) to (iii) below:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[17] A method of determining the malignancy of a tumor cell, comprising evaluating the localization of any polypeptide selected from among (1) to (3) below in the tumor cell, and determining the malignancy of the tumor cell on the basis of the presence or absence of the expression of the polypeptide in the cytoplasm:
   (1) a polypeptide comprising the amino acid sequence depicted in SEQ ID NO:2;
   (2) a polypeptide comprising an amino acid sequence having an identity of 90% or more to the amino acid sequence depicted in SEQ ID NO:2, and possessing an activity to enhance the motility or invasive potential of a cell when expressed in the cell; and
   (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell.
[18] The method described in [17], wherein the tumor is spindle cell sarcoma or colorectal cancer.
[19] An agent for determining the malignancy of a tumor cell, comprising an antibody that specifically recognizes a polypeptide consisting of the amino acid sequence depicted in SEQ ID N0:2.
[20] The agent described in [19], wherein the tumor is spindle cell sarcoma or colorectal cancer.
[21] An agent for inhibiting sarcoma metastasis comprising any polynucleotide selected from among (a) to (d) below or an expression vector capable of expressing the polynucleotide:
   (a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
   (b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell, and
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
   (d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.
[22] An agent for inhibiting the motility or invasive potential of a sarcoma cell, comprising any polynucleotide selected from among (a) to (d) below or an expression vector capable of expressing the polynucleotide:
   (a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
   (b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell, and
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide comprising the amino acid sequence depicted in SEQ ID NO:2 in the hybridized state; and
   (d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.
[23] A method of screening for a substance capable of inhibiting the metastasis of a sarcoma or the motility or invasive potential of a sarcoma cell, comprising the following steps of:
   (I) bringing into contact with each other a test substance and a cell that permits a measurement of the expression of any polypeptide or polynucleotide selected from among (1) to (5) below:
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell,
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell,
      (4) a polynucleotide comprising a coding region in the nucleotide sequence depicted in SEQ ID NO:1, and
      (5) a polynucleotide comprising a nucleotide sequence that encodes any polypeptide selected from among (1) to (3) above;
   (II) measuring the amount of any polypeptide or polynucleotide selected from among (1) to (5) above expressed in the cell contacted with the test substance, and comparing this amount expressed with the amount of the above-described polypeptide or polynucleotide expressed in a control cell not contacted with the test substance; and
   (III) selecting a test substance that suppresses the amount of any polypeptide or polynucleotide selected from among (1) to (5) above expressed, as a substance capable of inhibiting the metastasis of a sarcoma or the motility or invasive potential of a sarcoma cell, on the basis of the results of the comparison in (II) above.
[24] An agent for inhibiting the metastasis of a sarcoma, comprising any polypeptide selected from among (1) to (3) below or an expression vector capable of expressing the polypeptide:
   (1) a polypeptide comprising the amino acid sequence depicted in SEQ ID NO:4;
   (2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
   (3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.
[25] An agent for inhibiting the motility of a sarcoma cell, comprising any polypeptide selected from among (1) to (3) below or an expression vector capable of expressing the polypeptide:
   (1) a polypeptide comprising the amino acid sequence depicted in SEQ ID NO:4;
   (2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
   (3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.
[26] A method of screening for a peptide capable of inhibiting the metastasis of a sarcoma or the motility of a sarcoma cell, comprising the following steps of:
   (I) expressing a test polypeptide comprising at least three consecutive amino acids contained in the amino acid sequence depicted in SEQ ID NO:2 in a sarcoma cell expressing a polypeptide comprising the amino acid sequence depicted in SEQ ID NO:2;
   (II) measuring the motility of the cell in which the test polypeptide has been expressed, and comparing this motility with the motility of a control sarcoma cell in which the test polypeptide has not been expressed;
   (III) selecting a polypeptide that has suppressed the motility of the sarcoma cell as a polypeptide capable of inhibiting the metastasis of a sarcoma or the motility of a sarcoma cell, on the basis of the results of the comparison in (II) above.
[27] Any substance selected from among (i) to (iii) below for determining the risk of distant metastasis of a tumor:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[28] The substance described in [27], which is any one selected from among (ia) to (iiia) below:
   (ia) an antibody that specifically recognizes a polypeptide consisting of the amino acid sequence depicted in SEQ ID NO:2,
   a recognition epitope for the antibody being present within the region shown by amino acid numbers 1 to 385 in SEQ ID NO:2;
   (iia) a nucleic acid probe or primer capable of specifically detecting a polynucleotide consisting of the nucleotide sequence shown by nucleotide numbers 1 to 1229 in SEQ ID NO:1; and
   (iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.
[29] The substance described in [27] or [28], wherein the tumor is spindle cell sarcoma or colorectal cancer.
[30] Any substance selected from among (i) to (iii) below for determining the prognosis for survival of a tumor patient:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence shown by SEQ ID NO:2.
[31] The substance described in [30], wherein the substance is any one selected from among (ia) to (iiia) below:
   (ia) an antibody that specifically recognizes a polypeptide consisting of the amino acid sequence depicted in SEQ ID NO:2,
   a recognition epitope for the antibody being present within the region shown by amino acid numbers 1 to 385 in SEQ ID NO:2;
   (iia) a nucleic acid probe or primer capable of specifically detecting a polynucleotide consisting of the nucleotide sequence shown by nucleotide numbers 1 to 1229 in SEQ ID NO:1; and
   (iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.
[32] The substance described in [30] or [31], wherein the tumor is spindle cell sarcoma or colorectal cancer.
[33] Any substance selected from among (i) to (iii) below, for detecting colorectal cancer cells in colorectal mucosal tissue:
   (i) an antibody that specifically recognizes a polypeptide consisting of the amino acid sequence depicted in SEQ ID NO:2;
   (ii) a nucleic acid probe or primer capable of specifically detecting a polynucleotide consisting of the nucleotide sequence depicted in SEQ ID NO:1; and
   (iii) a nucleic acid probe or primer capable of specifically detecting a polynucleotide consisting of a nucleotide sequence that encodes a polypeptide consisting of the amino acid sequence depicted in SEQ ID NO:2.
[34] An antibody that specifically recognizes a polypeptide consisting of the amino acid sequence depicted in SEQ ID NO:2, for determining the malignancy of a tumor cell.
[35] The antibody described in [34], wherein the tumor is spindle cell sarcoma or colorectal cancer.
[36] Any polynucleotide selected from among (a) to (d) below or an expression vector capable of expressing the polynucleotide, for inhibiting the metastasis of a sarcoma:
   (a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
   (b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell, and
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
   (d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.
[37] Any polynucleotide selected from among (a) to (d) below or an expression vector capable of expressing the polynucleotide, for inhibiting the motility or invasive potential of a sarcoma cell:
   (a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
   (b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell, and
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
   (d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.
[38] Any polypeptide selected from among (1) to (3) below or an expression vector capable of expressing the polypeptide, for inhibiting the metastasis of a sarcoma:
   (1) a polypeptide comprising the amino acid sequence depicted in SEQ ID NO:4;
   (2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
   (3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.
[39] Any polypeptide selected from among (1) to (3) below or an expression vector capable of expressing the polypeptide, for inhibiting the motility of a sarcoma cell:
   (1) a polypeptide comprising the amino acid sequence depicted in SEQ ID NO:4;
   (2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
   (3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.
[40] A method for inhibiting the metastasis of a sarcoma in a mammal, comprising administering to the mammal an effective amount of any polynucleotide selected from among (a) to (d) below or an expression vector capable of expressing the polynucleotide:
   (a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
   (b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell, and
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
   (d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.
[41] A method for inhibiting the motility or invasive potential of a sarcoma cell in a mammal, comprising administering to the mammal an effective amount of any polynucleotide selected from among (a) to (d) below or an expression vector capable of expressing the polynucleotide:
   (a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
   (b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
      (1) a polypeptide comprising the amino acid sequence depicted in SEQ ID NO:2,
      (2) a polypeptide comprising an amino acid sequence having an identity of 90% or more to the amino acid sequence depicted in SEQ ID NO:2, and possessing an activity to enhance the motility or invasive potential of a cell when expressed in the cell, and
      (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
   (c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
   (d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.
[42] A method for inhibiting the metastasis of a sarcoma in a mammal, comprising administering to the mammal an effective amount of any polypeptide selected from among (1) to (3) below or an expression vector capable of expressing the polypeptide:
   (1) a polypeptide comprising the amino acid sequence depicted in SEQ ID NO:4;
   (2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
   (3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.
[43] A method for inhibiting the motility of a sarcoma cell in a mammal, comprising administering to the mammal an effective amount of any polypeptide selected from among (1) to (3) below or an expression vector capable of expressing the polypeptide:
   (1) a polypeptide comprising the amino acid sequence depicted in SEQ ID NO:4;
   (2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
   (3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.

### Advantages

Using the method of the present invention, it is possible to quickly determine the risk of onset of distant metastasis or the prognosis for survival in a tumor such as spindle cell sarcoma or colorectal cancer using a convenient technique such as immunological staining using an antibody or quantitative RT-PCR using a specific probe. Using the method of the present invention, it is possible to administer a selective treatment to the patient on the basis of the level of risk of onset of distant metastasis or the determination of prognosis for survival, thus improving the quality of tumor treatment. Specifically, for patients judged to have a poor prognosis using the method of the present invention, an improvement of the prognosis can be aimed at by proactively performing an adjuvant therapy, and attempting for early detection of metastatic foci by regular diagnostic imaging. Conversely, for patients judged to have a good prognosis using the method of the present invention, unnecessary adjuvant therapy can be avoided. In particular, because the method of the present invention is applicable to all types of spindle cell sarcoma, the prognosis for survival can be accurately determined even if a pathologic diagnosis is unestablished.
Using the method of the present invention, the presence or absence of colorectal cancer cells and the malignancy of colorectal cancer cells can be determined with high accuracy.
Using the agent for inhibiting metastasis of the present invention, it is possible to selectively suppress the metastasis of spindle cell sarcoma and improve the prognosis for survival in the spindle cell sarcoma. The agent for inhibiting metastasis of the present invention is distinctive in that the onset of distant metastasis, which is a factor that determines the prognosis of spindle cell sarcoma, is targeted. Because the expression levels in normal tissue are much lower than those in tumor tissue, the adverse reactions caused by expression suppressing treatment with an siRNA are expected to be very mild, and the treatment enables an effective treatment of spindle cell sarcoma when combined with currently available therapeutic methods.

### Brief Description of the Drawings

Fig. 1 summarizes a cDNA microarray analysis. RNAs extracted from spindle cell sarcoma and MSC were amplified using T7, and thereafter labeled at the time of reverse transcription. Competitive hybridization was performed on 23040 kinds of genes, and the expression level of each gene was converted to a numerical value.
Fig. 2 shows a search for genes associated with the malignancy. 65 patients were divided into two groups according to the presence or absence of each of the three events of recurrence, distant metastasis, and tumor-related death; C7059 was identified as a gene whose expression level differed between the two groups.
Fig. 3 shows the expression of the C7059 gene in a cDNA microarray: patients were divided into two groups according to onset of distant metastasis; the C7059 expression levels in the individual patients are shown. It is understood that many in the group of patients with onset of metastasis were positive for the expression of C7059, irrespective of pathologic diagnosis.
Fig. 4 shows a multivariate analysis. With tumor-related death as an endpoint, a multivariate analysis was performed on nine factors including C7059.
Fig. 5 shows genome and protein information on C7059.
Fig. 6 shows the genome structures of C7059 and ctC7059.
Fig. 7 shows the primary structure of the C7059 protein. Individual functional domains are shown. Being the shorter form, ctC7059 retains the protein-protein binding domain at the C-terminal.
Fig. 8 shows measurements of the amounts of C7059 and ctC7059 expressed using quantitative RT-PCR. In the sarcoma cell line U20S, which was used as the subject, the expression of ctC7059 was about 1/10 of that of C7059; it is understood that in spindle cell sarcoma, the expression of C7059 is predominant.

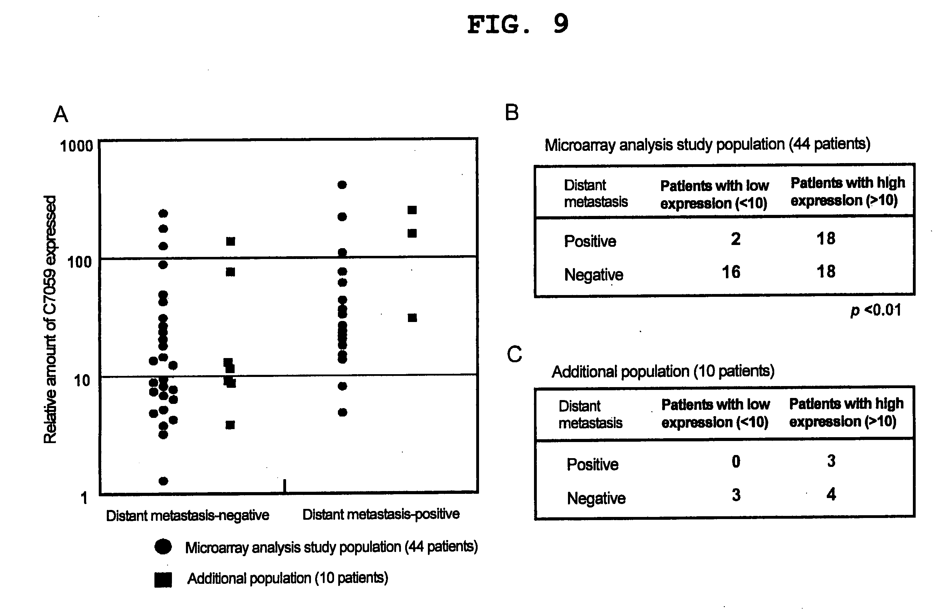

Fig. 9 shows a verification of microarray data using quantitative RT-PCR. A; C7059 expression level data by quantitative RT-PCR. With the amount expressed in MSC taken as 1, C7059 expression levels were converted to numerical values. In addition to the patients used in the microarray (solid circles, 44 patients), additional patients (solid squares, 10 patients) were analyzed. B; The correlation between C7059 expression level and onset of distant metastasis in the patients used in the microarray, divided into two groups with the relative expression level of 10 as the cutoff value. A significant difference in incidence was observed between the two groups. C; An analysis on the additional patients. It is understood that the incidence of distant metastasis was high in the patients with a relative expression level of 10 or more.
Fig. 10 shows how a C7059-specific polyclonal antibody was generated. A; Domestic rabbits were immunized with a fusion protein of 79 amino acids in an amino terminal region highly specific for C7059, and not contained in ctC7059, and GST, as the antigen, to generate a polyclonal antibody. B; An about 110 kD protein was recognized by Western blot. The expression level agreed with the C7059 mRNA level.
Fig. 11 shows immunocytological staining. After a C7059 expression vector was introduced into a C7059-negative sarcoma cell, the cell was stained using an antibody. It is seen that when C7059 was stained green with FITC, and actin stained red with Phalloidin, both were co-localized in some portions, with aggregation particularly in a portion where the morphology of lamellipodia was exhibited.
Fig. 12 shows immunocytological staining. The KS976 patient, who experienced pulmonary metastasis, was strongly positive for C7059 expression mainly in the cytoplasm, and also positive in the nucleus, whereas in the KS991 patient, who was negative for distant metastasis, stainability was observed in the nucleus only.
Fig. 13 shows induction of C7059 expression by introduction of an expression vector and suppression of C7059 expression with an siRNA. A; C7059 was integrated into a plasmid vector (PCAGGS), and the vector was introduced into a C7059-negative sarcoma cell, whereby good expression of C7059 was obtained. B; Four kinds of C7059-specific siRNAs were prepared. In particular, siRNAl-3 had a remarkable suppressive effect on C7059 expression at the mRNA and protein levels.
Fig. 14 shows the relationship between the expression of C7059 and the motility or invasive potential of cells. A; With induction of C7059 expression, the invasive potential was enhanced. B; With inhibition of C7059 expression, the motility and invasive potential decreased.
Fig. 15 shows a functional analysis of immortalized MSCs stably expressing C7059. With C7059 expression, both the motility (A) and invasive potential (B) was enhanced remarkably. C; With C7059 expression, the immortalized MSCs acquired an anchorage-independent growth potential in agar medium.
Fig. 16 shows an analysis of the binding of C7059 and the Src protein by co-immunoprecipitation. By performing immunoprecipitation with anti-FLAG antibody and Western blot with anti-Src antibody, the Src protein was detected (2). Conversely, by performing immunoprecipitation with anti-Src antibody and Western blot with anti-C7059 antibody (3) or anti-FLAG antibody (4), the C7059 protein was detected. Hence, it was found that C7059 and Src were bound together in cells.
Fig. 17 shows the confirmation of the co-localization of C7059 and Src by immunocytological staining. In immortalized MSCs expressing C7059 stably, C7059 was stained green with AF488, and Src stained red with AF555. It is understood that both were co-localized in some portions, with aggregation particularly in the lamellipodia.
Fig. 18 shows a functional analysis of ctC7059. A; ctC7059 was integrated into a plasmid vector (pCAGGS) to induce transient expression. B; When ctC7059 was expressed in C7059-positive sarcoma cells, the cell motility decreased.
Fig. 19 shows the expression of the C7059 protein in normal mucosa and polyps. In the polyps, C7059 expression was observed in the cell nucleus, but not observed in the cytoplasm.
Fig. 20 shows a patient 1 (Stage IV). The expression of the C7059 protein in normal mucosa and colorectal cancer is shown. The expression of the C7059 protein was observed in the cytoplasm of colorectal cancer cells, but not observed in the normal mucosa.
Fig. 21 shows a patient 2 (Stage III). A patient with hepatic metastasis and cerebral metastasis. The expression of the C7059 protein in normal mucosa and colorectal cancer is shown. The expression of the C7059 protein was observed in the cytoplasm of colorectal cancer cells, but not observed in the normal mucosa.
Fig. 22 shows a patient 3 (Stage IV). The expression of the C7059 protein in normal mucosa and colorectal cancer is shown. The expression of the C7059 protein was observed in the cytoplasm of colorectal cancer cells, but not observed in the normal mucosa.
Fig. 23 shows a patient 4 (Stage IV). The expression of the C7059 protein in normal mucosa and colorectal cancer is shown. The expression of the C7059 protein was predominant in the nucleus at portion of low heterogeneity, but predominant in the cytoplasm at invasive fronts. In the normal mucosa, no expression of the C7059 protein was observed.
Fig. 24 shows detection of the expression of C7059 in normal mucosa (N) and rectal or colic cancer (T) by RT-PCR. BACT: β actin.
Fig. 25 shows the correlation between disease-free survival rate and C7059 expression level (0 to 2) in Stage III rectal or colic cancer patients.
Fig. 26 shows the correlation between disease-free survival rate and C7059 expression level (0 to 2) in patients undergoing therapeutic surgery for extirpation of the colon. Best Mode for Carrying out the Invention

### 1. Method of determining the risk of onset of distant metastasis of tumor or the prognosis for survival of tumor patient

The present invention provides a method of determining the risk of distant metastasis of a tumor and the prognosis for survival of a tumor patient, comprising measuring the expression level of any polypeptide or polynucleotide selected from among (1) to (5) below in a tumor tissue, and determining the risk of distant metastasis of the tumor or the prognosis for survival of the tumor patient on the basis of the correlation between the expression level and the incidence of distant metastasis or the prognosis for survival of the tumor patient (determination method (I) of the present invention):
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell;
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(4) a polynucleotide comprising a coding region in the nucleotide sequence depicted in SEQ ID NO:1; and
(5) a polynucleotide comprising a nucleotide sequence that encodes any polypeptide selected from among (1) to (3) above.

Herein, the choice of tumor is not particularly limited. As the tumor, a sarcoma, colorectal cancer and the like can be mentioned. The sarcoma is preferably spindle cell sarcoma. The colorectal cancer is preferably a cancer that has developed in the colon or rectum. The determination method (I) of the present invention is suitably used for spindle cell sarcoma or colorectal cancer.

The subject genes whose expression levels are measured n the determination method (I) of the present invention are herein generically referred to as "sarcopodin". Specifically, in the determination method (I) of the present invention, the expression level of sarcopodin in a tumor tissue is measured. The sarcopodin used in the present invention is normally of human derivation. Specifically, the polypeptides (1) to (3) above are human sarcopodin polypeptides, and the polynucleotides (4) and (5) above are polynucleotides comprising a nucleotide sequence that encodes a human sarcopodin polypeptide.

The amino acid sequence and nucleotide sequence itself of human sarcopodin have been registered with GenBank under accession number AK074185 (as a polypeptide, FLJ00258) and the like, and the putative genome structures thereof have been clarified; however, no report is available on the biological function or clinical medical significance thereof. The present inventors have elucidated the biological function and clinical medical significance of sarcopodin, and provide the present invention on the basis of the achievement.

The sarcopodin polypeptides used in the present invention possess an activity to enhance the motility or invasive potential of a cell when expressed in the cell. The cell is normally derived from a mammal (for example, rat, mouse, guinea pig, rabbit, sheep, horse, swine, bovine, monkey, human; preferably human). The cell may be a tumor cell or a non-tumor cell. The cell is preferably a mesenchymal cell. Hence, as preferable tumor cells, sarcoma cells can be mentioned. As preferable non-tumor cells, mesenchymal stem cells can be mentioned.

The presence or absence of such activity can be determined by introducing an expression vector capable of expressing a desired polypeptide into a cell not expressing sarcopodin (preferably a sarcoma cell such as Saos2), and comparing the motility or invasive potential of the thus-obtained cell with that of a negative control cell.
Motility means the ability of cells to move actively. Invasive potential means the ability of cells to destroy the extracellular matrix and invade the tissue. The motility of cells can be measured according to, for example, the gold colloid method. The gold colloid method is a method wherein cells are seeded onto a cover glass with colloidal gold adhering thereto, and the area of the gold-lacking portion formed on the motion track thereof is calculated, whereby the motility of the cells is measured. The motility and invasive potential of cells can be evaluated by Boyden chamber assay and the like. In Boyden chamber assay, the motility is first evaluated by the number of cells that have passed an uncoated porous PET membrane, then using a PET membrane coated with a basal membrane, the number of cells that have degraded the basal membrane, passed the pores, and migrated to below the membrane is measured, and the ratio to the number of cells that have migrated is measured, whereby the invasive potential of the cells is evaluated.

The amino acid sequence contained in the polypeptide (2) above is an amino acid sequence having an identity of 90% or more, preferably 95% or more, more preferably 98% or more, still more preferably 99% or more, to the amino acid sequence depicted in SEQ ID NO:2.

Here, "identity" means a ratio (%) of identical amino acid residues to all overlapping amino acid residues in the optimal alignment where two amino acid sequences are aligned using a mathematical algorithm known in the technical field (preferably, the algorithm can consider introduction of gaps on one or both of the sequences for the best alignment).

Amino acid sequence identity in the present description can be calculated using the homology calculation algorithm NCBI BLAST-2 (National Center for Biotechnology Information Basic Local Alignment Search Tool) under the following conditions (matrix=BLOSUM62; gap open=11; gap extension=1; x_ dropoff=50; expectancy=10; filtering=ON). Algorithms to determine amino acid sequence identity include, for example, but are not limited to, the algorithm described in Karlin et al., Proc. Natl. Acad. Sci. USA, 90:5873-5877 (1993) [the algorithm is incorporated in the NBLAST and XBLAST programs (version 2.0) (Altschul et al., Nucleic Acids Res., 25:3389-3402 (1997))], the algorithm described in Needleman et al., J. Mol. Biol., 48:444-453 (1970) [the algorithm is incorporated in the GAP program in the GCG software package], the algorithm described in Myers and Miller, CABIOS, 4:11-17 (1988) [the algorithm is incorporated in the ALIGN program (version 2.0), which is part of the CGC sequence alignment software package], the algorithm described in Pearson et al., Proc. Natl. Acad. Sci. USA, 85:2444-2448 (1988) [the algorithm is incorporated in the FASTA program in the GCG software package] and the like.

The amino acid sequence contained in the polypeptide (3) above is an amino acid sequence resulting from deletion, substitution, insertion or addition of one or a plurality of amino acids in the amino acid sequence depicted in SEQ ID NO:2, for example, (1) an amino acid sequence resulting from deletion of one or a plurality of (preferably 1 to 30, more preferably 1 to 10, still more preferably one to several (2 to 5)) amino acids in the amino acid sequence depicted in SEQ ID NO:2, (2) an amino acid sequence resulting from addition of one or a plurality of (preferably 1 to 30, more preferably 1 to 10, still more preferably one to several (2 to 5)) amino acids in the amino acid sequence depicted in SEQ ID NO:2, (3) an amino acid sequence resulting from insertion of one or a plurality of (preferably 1 to 30, more preferably 1 to 10, still more preferably one to several (2 to 5)) amino acids in the amino acid sequence depicted in SEQ ID NO:2, (4) an amino acid sequence resulting from substitution of one or a plurality of (preferably 1 to 30, more preferably 1 to 10, still more preferably one to several (2 to 5)) amino acids in the amino acid sequence depicted in SEQ ID NO:2 by other amino acids, or (5) an amino acid sequence being a combination of the mutations (1) to (4) above (in this case, the total number of mutated amino acids is preferably 1 to 30, more preferably 1 to 10, still more preferably one to several (2 to 5)).

In one preferred embodiment, the polypeptides (1) to (3) above are natural human sarcopodin polypeptides. "Natural" refers to the existence of an amino acid sequence that constitutes a polypeptide. Natural human sarcopodin amino acid sequences include, but are not limited to, the amino acid sequence depicted in SEQ ID NO:2. It is known that a polymorphism (individual differences) usually exists in gene-constituting nucleotide sequences. Polypeptides having an amino acid sequence different from the amino acid sequence depicted in SEQ ID NO:2, resulting from such polymorphism of the sarcopodin gene, are also included in natural human sarcopodin polypeptides, as far as they possess an activity to enhance the motility or invasive potential of a cell when expressed in the cell.

The polynucleotides (4) and (5) above are DNAs or RNAs, and are preferably RNAs (more preferably mRNAs). As the mRNAs, mature mRNAs and initial transcription products (immature mRNAs) that produce mature mRNAs by post-transcriptional processing can be mentioned. The mRNAs are preferably mature mRNAs. Hence, the polynucleotides (4) and (5) above can be mature mRNAs of sarcopodin. As the polynucleotide of (4) above, a polynucleotide comprising the nucleotide sequence depicted in SEQ ID NO:1 can be mentioned. Nucleotide sequences are herein described as DNA sequences unless otherwise specified; however, when the polynucleotide is an RNA, thymine (T) should read as uracil (U) as appropriate.

In the determination method (I) of the present invention, the expression level of a sarcopodin polypeptide or a polynucleotide (for example, mRNA) that encodes the polypeptide in a tumor tissue separated from the patient who is the subject of measurement is measured.

The expression level of a sarcopodin polypeptide can be measured using an antibody that specifically recognizes sarcopodin, for example,
(i) an antibody that specifically recognizes a polypeptide consisting of the amino acid sequence depicted in SEQ ID NO:2, by an immunological technique. As the immunological technique, flowcytometry analysis, radioisotope immunoassay (RIA), ELISA (Methods in Enzymol. 70: 419-439 (1980)), Western blotting, immunohistochemical staining and the like can be mentioned.

An antibody that specifically recognizes sarcopodin can be produced by a known ordinary method of production using a sarcopodin polypeptide or a partial peptide thereof having the antigenicity as an immunogen. As mentioned herein, antibodies include, but are not limited to, natural type antibodies such as polyclonal antibodies and monoclonal antibodies (mAbs), chimeric antibodies, humanized antibodies, and single-stranded antibodies that can be produced using gene recombination technology, and binding fragments thereof. Preferably, the antibody is a polyclonal antibody, a monoclonal antibody or a binding fragment thereof. A binding fragment means a partial region of one of the above-described antibodies having the specific binding activity; specifically, for example, F(ab')₂, Fab', Fab, Fv, sFv, dsFv, sdAb and the like can be mentioned (Exp. Opin. Ther. Patents, Vol.6, No.5, p.441-456, 1996). The class of antibody is not particularly limited; antibodies of any itotypes such as IgG, IgM, IgA, IgD and IgE are encompassed. Preferably, the class is IgG or IgM, and in view of the ease of purification and the like, the class is more preferably IgG.

The expression level of a polynucleotide that encodes a sarcopodin polypeptide can be measured by a method known per se, using a nucleic acid probe or primer capable of specifically detecting the polynucleotide, for example,
(ii) a nucleic acid probe or primer capable of specifically detecting a polynucleotide consisting of the nucleotide sequence depicted in SEQ ID NO:1, or
(iii) a nucleic acid probe or primer capable of specifically detecting a polynucleotide consisting of a nucleotide sequence that encodes a polypeptide consisting of the amino acid sequence depicted in SEQ ID NO:2. As examples of the measuring method, RT-PCR, Northern blotting, in situ hybridization, cDNA array and the like can be mentioned.

In one preferred embodiment, the nucleic acid probe used in the determination method (I) of the present invention is a polynucleotide comprising a nucleotide sequence of about 15 bases or more, preferably about 18 to about 500 bases, more preferably about 18 to about 200 bases, still more preferably about 18 to about 50 bases, in continuity, or a complementary sequence for the same, contained in the nucleotide sequence depicted in SEQ ID NO:1 or a nucleotide sequence that encodes a polypeptide consisting of the amino acid sequence depicted in SEQ ID NO:2.

In another preferred embodiment, the nucleic acid probe used in the determination method (I) of the present invention is a polynucleotide comprising a nucleotide sequence that hybridizes with the nucleotide sequence depicted in SEQ ID NO:1 or a nucleotide sequence that encodes a polypeptide consisting of the amino acid sequence depicted in SEQ ID NO:2 under stringent conditions. The hybridization can be performed by a method known per se or a method based thereon, for example, a method described in Molecular Cloning, 2nd edition (J. Sambrook et al., Cold Spring Harbor Lab. Press, 1989) and the like. Examples of stringent conditions include those wherein a hybridization reaction at 45°C in 6×SSC (sodium chloride/sodium citrate) is followed by one time or more of washing at 65°C in 0.2×SSC/0.1% SDS and the like. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate. The length of the nucleic acid probe is normally about 15 bases or more, preferably about 18 to about 500 bases, more preferably about 18 to about 200 bases, still more preferably about 18 to about 50 bases.

The nucleic acid probe may comprise an additional sequence (a nucleotide sequence not complementary to the polynucleotide which is the target of detection), as far as the specific detection is not interfered with.
The nucleic acid probe may be labeled with an appropriate labeling agent, for example, a radioisotope (e.g., ¹²⁵I, ¹³¹I, ³H, ¹⁴C, ³²P, ³³P and the like), enzyme (e.g., β-galactosidase, β-glucosidase, alkaline phosphatase, peroxidase, malate dehydrogenase and the like), fluorescent substance (e.g., fluorescamine, fluorescein isothiocyanate and the like), luminescent substance (e.g., luminol, luminol derivative, luciferin, lucigenin and the like) and the like. Alternatively, in the vicinity of a fluorescent substance (e.g., FAM, VIC and the like), a quencher (quenching substance) that absorbs the fluorescence energy produced by the fluorescent substance may further be bound. In this mode of embodiment, upon the detection reaction, the fluorescent substance and the quencher become separated from each other and fluorescence is detected.

The nucleic acid primer used in the determination method (I) of the present invention may be any one, as far as it has been designed to enable specific amplification of a portion or the entire region of a polynucleotide consisting of the nucleotide sequence depicted in SEQ ID NO:1 or a nucleotide sequence that encodes a polypeptide consisting of the amino acid sequence depicted in SEQ ID NO:2. For example, a pair of polynucleotides can be mentioned which is a combination of a polynucleotide comprising a nucleotide sequence as long as about 15 to about 50 bases, preferably about 18 to about 30 bases, which hybridizes with a portion of a complementary sequence for the above-described nucleotide sequence, and a polynucleotide comprising a nucleotide sequence as long as about 15 to about 50 bases, preferably about 18 to about 30 bases, which hybridizes with a portion of the above-described nucleotide sequence on the 3' side from said hybridization site, wherein the lengths of nucleic acid fragments amplified thereby are about 50 to about 1,000 bases, preferably about 50 to about 500 bases, more preferably about 50 to about 200 bases.

The nucleic acid primer may comprise an additional sequence (a nucleotide sequence not complementary to the polynucleotide which is the target of detection), as far as the specific detection is not interfered with.
The nucleic acid primer may be labeled with an appropriate labeling agent, for example, a radioisotope (e.g., ¹²⁵I, ¹³¹I, ³H, ¹⁴C, ³²P, ³³P and the like), enzyme (e.g., β-galactosidase, β-glucosidase, alkaline phosphatase, peroxidase, malate dehydrogenase and the like), fluorescent substance (e.g., fluorescamine, fluorescein isothiocyanate and the like), luminescent substance (e.g., luminol, luminol derivative, luciferin, lucigenin and the like) and the like.

The nucleic acid probe or primer may be a DNA or an RNA, and may be single-stranded or double-stranded. In the case of a double strand, the nucleic acid may be a double-stranded DNA, a double-stranded RNA, or a DNA/RNA hybrid. Therefore, when a nucleic acid having a certain nucleotide sequence is described herein, it should be understood that the term nucleic acid is used with a meaning that encompasses all of single-stranded polynucleotides having the nucleotide sequence, single-stranded polynucleotides having a sequence complementary to the nucleotide sequence, and double-stranded polynucleotides which are hybrids thereof unless otherwise stated.
The above-described nucleic acid probe or primer can be synthesized, for example, using an automated DNA/RNA synthesizer according to a conventional method, on the basis of information on the nucleotide sequence depicted in SEQ ID NO:1.

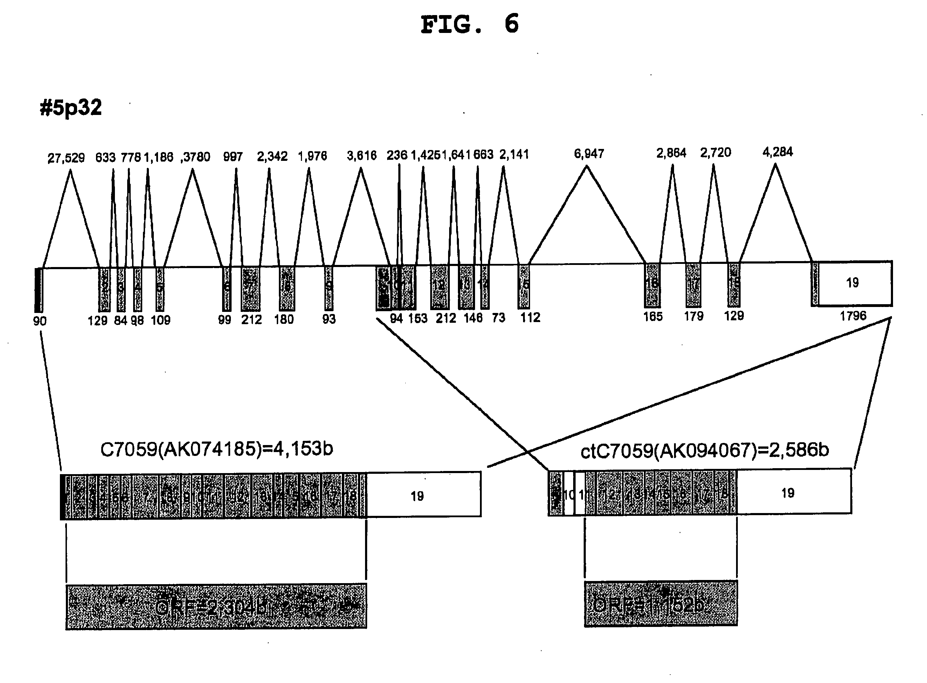

The sarcopodin gene has a second transcription initiation point in the 9th intron thereof; this second transcription produces the shorter form, which has a structure wherein the N-terminal region of the sarcopodin described above (hereinafter sometimes referred to as the longer form) is lacked (Fig. 6). For example, as a shorter form human sarcopodin, a polypeptide consisting of the amino acid sequence depicted in SEQ ID NO:4 encoded by the nucleotide sequence depicted in SEQ ID NO:3 (coding region: nucleotide numbers 317 to 1468) can be mentioned. This shorter form human sarcopodin lacks 385 amino acids at the N-terminus of the human sarcopodin consisting of the amino acid sequence depicted in SEQ ID NO:2, and consists of the remainder (the amino acid sequence shown by amino acid numbers 386 to 768 in SEQ ID NO:2). Therefore, in the determination method (I) of the present invention, when an antibody that specifically recognizes a region shared by the longer form and the shorter form of sarcopodin, or a nucleic acid probe or primer that specifically detects a shared region, is used, the sum of the amount of the longer and shorter forms expressed will be measured. Such cases also fall within the scope of the present invention, as far as the resulting expression level measured includes the expression level of any polypeptide or polynucleotide selected from among (1) to (5) above.

As shown in Examples below, the expression of the longer form is predominant over the expression of the shorter form in tumors (particularly spindle cell sarcoma). Therefore, by specifically detecting the longer form of sarcopodin, the risk of distant metastasis or prognosis for survival in a tumor can be determined with higher sensitivity. Hence, in one preferred embodiment, of the antibodies, nucleic acid probes or primers in (i) to (iii) above, one capable of specifically detecting a region that is present in the longer form, but not present in the shorter form, is used in measuring the expression level. As such antibodies, nucleic acid probes and primers, (ia) to (iiia) below can be mentioned:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

Of them, the antibodies, nucleic acid probes or primers in (ib) to (iiib) below are preferable because they recognize a region having a sequence of particularly high specificity for longer-form sarcopodin.
(ib) an antibody that specifically recognizes a polypeptide consisting of the amino acid sequence depicted in SEQ ID NO:2, a recognition epitope for the antibody being present within the region shown by amino acid numbers 1 to 385 in SEQ ID NO:2;
(iib) a nucleic acid probe or primer capable of specifically detecting a polynucleotide consisting of the nucleotide sequence shown by nucleotide numbers 117 to 413 in SEQ ID NO:1; and
(iiib) a nucleic acid probe or primer capable of specifically detecting a polynucleotide consisting of a nucleotide sequence that encodes a polypeptide consisting of the amino acid sequence shown by amino acid numbers 35 to 113 in SEQ ID NO:2.

Next, on the basis of the expression level of sarcopodin measured, the risk of distant metastasis of a tumor or the prognosis for survival of a tumor patient is determined. As shown in Examples below, the higher the expression level of sarcopodin in a tumor tissue is, the higher the incidence of distant metastasis is, and the prognosis for survival of the patient is worse. The above-described determination is made on the basis of such a correlation between the expression level of sarcopodin and the incidence of distant metastasis of the tumor or the prognosis for survival of the tumor patient.

For example, tumors are separated from a patient having a good prognosis for survival without distant metastasis (negative control) and a patient having a poor prognosis for survival with distant metastasis (positive control), the sarcopodin expression levels in the tumors separated from the subject patients are compared with those of a positive control and a negative control. Alternatively, a diagram of the correlation between the sarcopodin expression level in the tumors and the incidence of distant metastasis or prognosis for survival (e.g., length of survival) may be prepared in advance, and the sarcopodin expression levels in the tumors separated from the subject patients may be compared with the diagram of the correlation. The comparison of the expression levels is preferably made on the basis of the presence or absence of a significant difference.

When the sarcopodin expression level in the subject of measurement is relatively high, judging from the results of the comparison of sarcopodin expression levels, it can be determined that the risk of onset of distant metastasis of a tumor is relatively high, or the prognosis for survival of the patient is relatively poor. Conversely, when the sarcopodin expression level in the subject of measurement is relatively low, it can be determined that the risk of onset of distant metastasis of a tumor is relatively low, or the prognosis for survival of the patient is relatively good.

The determination can also be made by previously setting a cutoff value for sarcopodin expression levels, and comparing the sarcopodin expression level measured and this cutoff value. For example, when the sarcopodin expression level measured is not less than the aforementioned cutoff value, it can be determined that the risk of onset of distant metastasis of a tumor is relatively high, or the prognosis for survival of the patient is relatively poor. Conversely, when the sarcopodin expression level is lower than the cutoff value, it can be determined that the risk of onset of distant metastasis of a tumor is relatively low, or the prognosis for survival of the patient is relatively good. "A cutoff value" is a value that meets the requirements for both high diagnostic sensitivity (true positive rate) and high diagnostic specificity (true negative rate) when a disease or condition is judged with that value as a criterion. For example, a sarcopodin expression level that produces high positive rates in subjects who have experienced distant metastasis or subjects whose survival is not longer than a given length, and high negative rates in subjects who have not experienced distant metastasis or subjects whose survival exceeds the given length, can be set as a cutoff value.

The present invention also provides an agent for determining the risk of distant metastasis of a tumor or the prognosis for survival of a tumor patient, containing any substance selected from among (i) to (iii) above (referred to as the agent (I) of the present invention). The substance is preferably any substance selected from among (ia) to (iiia) above, more preferably any substance selected from among (ib) to (iiib) above. The agent (I) of the present invention can be a kit for determining the risk of distant metastasis of a tumor or the prognosis for survival of a tumor patient. Using the agent (I) of the present invention, the risk of distant metastasis of a tumor or the prognosis for survival of a tumor patient can easily be determined by the above-described method.

The substances (i) to (iii) can be separately (or in a mixed form if possible) dissolved in water or an appropriate buffer solution (e.g., TE buffer, PBS and the like) to obtain an appropriate concentration, and preserved at about -20°C to 4°C.

The agent (I) of the present invention may further contain other ingredients necessary for the implementation of the method of measuring sarcopodin expression levels as constituents thereof according to the method.
For example, provided that the agent (I) of the present invention contains the substance (i) above, the risk of distant metastasis or prognosis for survival in spindle cell sarcoma can be determined by measuring the sarcopodin expression level by immunological technique. In this case, the agent (I) of the present invention can further contain a labeled secondary antibody, color developing substrate, blocking liquid, washing buffer solution, ELISA plate, blotting membrane and the like.
Provided that the agent (I) of the present invention contains the substance (ii) or (iii) above, the risk of distant metastasis of a tumor or the prognosis for survival of a tumor patient can be determined by measuring sarcopodin expression level by RT-PCR, Northern blotting, in situ hybridization, cDNA array and the like. When RT-PCR is used for the measurement, the agent (I) of the present invention may further contain a 10×PCR reaction buffer solution, 10×MgCl₂ aqueous solution, 10×dNTPs aqueous solution, Taq DNA polymerase (5 U/µL), reverse transcriptase and the like. When Northern blotting or cDNA array is used for the measurement, the agent (I) of the present invention can further contain a blotting buffer solution, labeling reagent, blotting membrane and the like. When in situ hybridization is used for the measurement, the agent (I) of the present invention can further contain a labeling reagent, color developing substrate and the like.

### 2. Method of detecting colorectal cancer cells

As shown in Examples below, in colorectal mucosal tissue, sarcopodin is little expressed in normal tissue and polyps, whereas in colorectal cancer cells, sarcopodin is specifically expressed. Therefore, by evaluating the expression of sarcopodin in colorectal mucosal tissue, and analyzing for the presence or absence of the expression thereof, colorectal cancer cells can be detected in colorectal mucosal tissue.

Accordingly, the present invention provides a method of detecting colorectal cancer cells in colorectal mucosal tissue, comprising evaluating the expression of any polypeptide or polynucleotide selected from among (1) to (5) below in colorectal mucosal tissue, and determining whether or not colorectal cancer cells are contained in the tissue on the basis of the presence or absence of the expression (detection method (I) of the present invention):
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell;
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(4) a polynucleotide comprising a coding region in the nucleotide sequence depicted in SEQ ID NO:1; and
(5) a polynucleotide comprising a nucleotide sequence that encodes any polypeptide selected from among (1) to (3) above.

The definitions for the polypeptides or polynucleotides (1) to (5) above are the same as those described in the (1. Method of determining the risk of onset of distant metastasis of tumor or the prognosis for survival of tumor patient) section.

In the detection method (I) of the present invention, the expression of a sarcopodin polypeptide or a polynucleotide (for example, mRNA) that encodes the polypeptide in colorectal mucosal tissue separated from a patient who is the subject of measurement is evaluated.

The expression of the sarcopodin polypeptide can be evaluated according to the method described in detail in the (1. Method of determining the risk of onset of distant metastasis of tumor or the prognosis for survival of tumor patient) section, using the antibody, nucleic acid probe, primer and the like described in (i) to (iii) above, preferably (ia) to (iiia) above, more preferably (ib) to (iiib) above.

Next, on the basis of the presence or absence of the expression of sarcopodin in the colorectal mucosal tissue, whether or not colorectal cancer cells are present in the tissue is determined.

When a cell expressing sarcopodin is found in the colorectal mucosal tissue from the subject of measurement, it can be determined that the probability of the presence of colorectal cancer cells in the tissue is high. Conversely, when no cell expressing sarcopodin is found in the colorectal mucosal tissue from the subject of measurement, it can be judged that the probability of the presence of colorectal cancer cells in the tissue is low.

The present invention also provides an agent for detecting colorectal cancer cells in colorectal mucosal tissue, containing any substance selected from among (i) to (iii) above (referred to as the agent (II) of the present invention). The substance is preferably any substance selected from among (ia) to (iiia) above, more preferably any substance selected from among (ib) to (iiib) above. The agent (II) of the present invention) can be a kit for detecting colorectal cancer cells in colorectal mucosal tissue. Using the agent (II) of the present invention, colorectal cancer cells in colorectal mucosal tissue can easily be detected by the above-described method.

The substances (i) to (iii) can be separately (or in a mixed form if possible) dissolved in water or an appropriate buffer solution (e.g., TE buffer, PBS and the like) to obtain an appropriate concentration, and preserved at about -20°C to 4°C.

The agent (II) of the present invention may further contain other ingredients necessary for the implementation of a method of evaluation of sarcopodin expression as constituents thereof according to the method.
For example, provided that the agent (II) of the present invention contains the substance (i) above, the presence or absence of colorectal cancer cells in colorectal mucosal tissue can be determined by evaluating the sarcopodin expression by an immunological technique. In this case, the agent (II) of the present invention can further contain a labeled secondary antibody, color developing substrate, blocking liquid, washing buffer solution, ELISA plate, blotting membrane and the like.
Provided that the agent (II) of the present invention contains the substance (ii) or (iii) above, the presence or absence of colorectal cancer cells in colorectal mucosal tissue can be determined by measuring sarcopodin expression level by RT-PCR, Northern blotting, in situ hybridization, cDNA array and the like. When RT-PCR is used for the measurement, the agent (I) of the present invention can further contain a 10×PCR reaction buffer solution, 10×MgCl₂ aqueous solution, 10×dNTPs aqueous solution, Taq DNA polymerase (5 U/µL), reverse transcriptase and the like. When Northern blotting or cDNA array is used for the measurement, the agent (II) of the present invention can further contain a blotting buffer solution, labeling reagent, blotting membrane and the like. When in situ hybridization is used for the measurement, the agent (II) of the present invention can further contain a labeling reagent, color developing substrate and the like.

### 3. Method of determining the malignancy of tumor cell

As shown in Examples below, in spindle cell sarcoma cells without distant metastasis and colorectal cancer cells of low heterogeneity, sarcopodin was localized in the nucleus, whereas in spindle cell sarcoma cells with distant metastasis and tumor cells of high heterogeneity, sarcopodin was also localized in the cytoplasm; it is realized that as the malignancy of tumor cells changes, the intracellular localization of sarcopodin changes. Therefore, by evaluating the localization of sarcopodin in a tumor cell, and analyzing for the presence or absence of the expression in the cytoplasm thereof, the malignancy of the tumor cell can be determined.

Accordingly, the present invention provides a method of determining the malignancy of a tumor cell, comprising evaluating the localization of any polypeptide selected from among (1) to (3) below in the tumor cell, and determining the malignancy of the tumor cell on the basis of the presence or absence of the expression of the polypeptide in the cytoplasm (determination method (II) of the present invention):
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell; and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell.

The definitions for the polypeptides (1) to (3) above are the same as those described in the (1. Method of determining the risk of distant metastasis of tumor or the prognosis for survival of tumor patient) section.

In the determination method (II) of the present invention, the localization of a sarcopodin polypeptide in a tumor cell contained in a tumor tissue separated from a patient who is the subject of measurement is evaluated.

The intracellular localization of the sarcopodin polypeptide can be evaluated using the antibody described in (i) above, preferably (ia) above, more preferably (ib) above, by immunohistochemical staining.

Next, the malignancy of a tumor cell is determined on the basis of the presence or absence of the expression of sarcopodin in the cytoplasm. When sarcopodin expression is found in the cytoplasm of a tumor cell from the subject of measurement, the tumor cell can be determined to have a high malignancy. Conversely, when sarcopodin expression is not observed in the cytoplasm of the tumor cell from the subject of measurement, but remains in the nucleus, or when sarcopodin expression is not observed in any site in the tumor cell from the subject of measurement, the tumor cell can be determined to have a low malignancy.

The present invention also provides an agent for determining the malignancy of a tumor cell, containing the antibody described in (i) above (referred to as the agent (III) of the present invention). The antibody is preferably the antibody described in (ia) above, more preferably the antibody described in (ib) above. The agent (III) of the present invention can be a kit for determining the malignancy of a tumor cell. Using the agent (III) of the present invention, the malignancy of a tumor cell can easily be determined by the method described above.

The antibody described in (i) above can be dissolved in water or an appropriate buffer solution (e.g., TE buffer, PBS and the like) to obtain an appropriate concentration, and preserved at about -20°C to 4°C.

The agent (III) of the present invention may further contain other ingredients necessary for the implementation of a method of evaluation of sarcopodin expression as constituents thereof according to the method. For example, the agent (III) of the present invention can further contain a labeled secondary antibody, color developing substrate, blocking liquid, washing buffer solution and the like that are useful in immunohistochemical staining.

### 4. Agent for inhibiting the metastasis of sarcoma or the motility or invasive potential of sarcoma cells

The present invention provides an agent for inhibiting the metastasis of a sarcoma or the motility or invasive potential of a sarcoma cell, containing a substance that inhibits the expression or function of sarcopodin (inhibitor
(I) of the present invention). In a preferred embodiment of the substance, the following polynucleotides can be mentioned:
   (A) a polynucleotide (DNA or RNA) comprising a nucleotide sequence complementary to the nucleotide sequence of an mRNA (mature mRNA or initial transcription product) that encodes sarcopodin or a partial sequence thereof as long as 15 bases or more; and
   (B) a polynucleotide (DNA or RNA) comprising a nucleotide sequence hybridizable with an mRNA (mature mRNA or initial transcription product) or chromosome DNA that encodes sarcopodin under physiological conditions for an animal (preferably human) which is the subject of treatment, and suppressing the transcription or translation of sarcopodin in the hybridized state.

The polynucleotides (A) and (B) above are specifically identified as follows:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
   (1) a polypeptide comprising the amino acid sequence depicted in SEQ ID NO:2,
   (2) a polypeptide comprising an amino acid sequence having an identity of 90% or more to the amino acid sequence depicted in SEQ ID NO:2, and possessing an activity to enhance the motility or invasive potential of a cell when expressed in the cell, and
   (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

The definitions for the polypeptides (1) to (3) above are the same as those described in the (1. Method of determining the risk of distant metastasis or prognosis for survival in spindle cell sarcoma, and agent therefor) section. The polynucleotides (a) to (d) above are useful as antisense nucleic acids or siRNAs against sarcopodin. Embodiments of the polynucleotides (a) to (d) above can be chosen as appropriate according to the intended use thereof.

"An antisense nucleic acid" refers to a nucleic acid comprising a nucleotide sequence hybridizable with a target mRNA (mature mRNA or initial transcription product) under physiological conditions for the cells that express the target mRNA, and being capable of inhibiting the translation of the polypeptide encoded by the target mRNA in the hybridized state. The antisense nucleic acid may be a DNA or an RNA, and may be a DNA/RNA chimera. Since natural-form antisense nucleic acids have the phosphodiester linkages thereof decomposed readily by nucleases being present in the cells, the antisense nucleic acid of the present invention can also be synthesized using a modified nucleotide such as the thiophosphate form (phosphate bond P=O is replaced with P=S) or the 2'-O-methyl form, which are stable to the nucleases. Other factors important for the design of the antisense nucleic acid include increasing the water solubility and cell membrane permeability and the like; these can also be achieved by improving dosage forms, such as the use of liposomes or microspheres.

The length of the portion that hybridizes with the target mRNA in the antisense nucleic acid is not particularly limited, as far as the portion is hybridizable specifically with a mature mRNA or initial transcription product of sarcopodin, and capable of inhibiting the translation of the sarcopodin polypeptide in the hybridized state; the length is about 15 bases for the shortest, and as long as the full-length sequence of the mRNA (mature mRNA or initial transcription product) for the longest. In view of hybridization specificity, the length of the portion that hybridizes with the target mRNA is, for example, about 15 bases or more, preferably about 18 bases or more, more preferably about 20 bases or more. In view of the ease of synthesis, antigenicity issues and the like, the length of the portion that hybridizes with the target mRNA is, for example, about 200 bases or less, preferably about 50 bases or less, more preferably about 30 bases or less. Hence, the length of the portion that hybridizes with the target mRNA is, for example, about 15 to about 200 bases, preferably about 18 to about 50 bases, more preferably about 20 to about 30 bases.

The target nucleotide sequence for the antisense nucleic acid is not particularly limited, as far as it is a sequence such that the translation of sarcopodin is inhibited when hybridizing with the antisense nucleic acid; the sequence may be the full-length sequence of an mRNA (mature mRNA or initial transcription product) of sarcopodin or a partial sequence thereof (for example, about 15 bases or more, preferably about 18 bases or more, more preferably about 20 bases or more), or an intron portion of the initial transcription product; however, when an oligonucleotide is used as the antisense nucleic acid, it is desirable that the target sequence be located between the 5'-end of the mRNA of sarcopodin and the C-terminal of the coding region.

The nucleotide sequence of the portion that hybridizes with the target mRNA in the antisense nucleic acid varies depending on the base composition of the target sequence, and has an identity of normally about 90% or more (preferably 95% or more, most preferably 100%) to the complementary sequence for the target sequence, so as to be capable of hybridizing with the sarcopodin mRNA under physiological conditions. Identity in nucleotide sequence can be calculated using, for example, the homology calculation algorithm NCBI BLAST-2 (National Center for Biotechnology Information Basic Local Alignment Search Tool) under the following conditions (gap open=5; gap extension=2; x_ dropoff=50; expectancy=10; filtering=ON).

The size of the antisense nucleic acid is normally about 15 bases or more, preferably about 18 bases or more, more preferably about 20 bases or more. In view of the ease of synthesis, antigenicity issues and the like, the size is normally about 200 bases or less, preferably about 50 bases or less, more preferably about 30 bases or less.

Furthermore, the antisense nucleic acid may be one not only capable of hybridizing with the sarcopodin mRNA or initial transcription product thereof to inhibit the translation, but also capable of binding to the sarcopodin gene, which is a double-stranded DNA, to form a triplex and inhibit the transcription into mRNA.

An siRNA against sarcopodin is a double-stranded oligo-RNA comprising a nucleotide sequence complementary to the nucleotide sequence of sarcopodin mRNA (mature mRNA or initial transcription product) or a partial sequence thereof (preferably in the coding region) (in the case of the initial transcription product, an intron portion is included). What is called RNA interference (RNAi), a phenomenon in which transferring a short double-stranded RNA into a cell results in the degradation of mRNAs that are complementary to the RNA, has long been known to occur in nematodes, insects, plants and the like; recently, this phenomenon was confirmed as occurring also in animal cells [Nature, 411(6836): 494-498 (2001)], and this is attracting attention as a ribozyme-substitute technique.

A representative siRNA is a double-stranded oligo-RNA consisting of an RNA having a sequence complementary to the nucleotide sequence of the mRNA of the target gene or a partial sequence thereof (hereinafter, target nucleotide sequence) and a complementary strand for the RNA. A single-stranded RNA wherein a sequence (first sequence) complementary to the target nucleotide sequence and a complementary sequence (second sequence) for the same are linked via a hairpin loop portion, and wherein the first sequence forms a double-stranded structure with the second sequence by assuming a hairpin loop structure (small hairpin RNA: shRNA), also represents a preferred embodiment of siRNA.

The length of the portion complementary to the target nucleotide sequence, contained in the siRNA, is normally about 15 bases or more, preferably 18 bases or more, more preferably about 20 bases (representatively about 21 to 23 bases long), but is not particularly limited, as far as RNA interference can be caused. When the siRNA is longer than 23 bases, the siRNA undergoes degradation in cells to produce an siRNA about 20 bases long; therefore, theoretically, the upper limit of the portion complementary to the target nucleotide sequence is the full length of the nucleotide sequence of an mRNA (mature mRNA or initial transcription product) of the target gene. However, in view of the ease of synthesis, antigenicity issues and the like, the length of the complementary portion is, for example, about 200 bases or less, preferably about 50 bases or less, more preferably about 30 bases or less. Hence, the length of the complementary portion is, for example, about 15 bases or more, preferably about 18 to about 200 bases, more preferably about 20 to about 50 bases, still more preferably about 20 to about 30 bases.

The length of the siRNA is normally about 18 bases or more, for example, about 20 bases (representatively about 21 to 23 bases long), but is not particularly limited, as far as RNA interference can be caused; there is theoretically no upper limit on the length of the siRNA. However, in view of the ease of synthesis, antigenicity issues and the like, the length of the siRNA is, for example, about 200 bases or less, preferably about 50 bases or less, more preferably about 30 bases. Hence, the length of the siRNA is, for example, about 18 bases or more, preferably about 18 to about 200 bases, more preferably about 20 to about 50 bases, still more preferably about 20 to about 30 bases. The length of the shRNA is expressed as the length of the double-stranded moiety when the shRNA assumes a double-stranded structure.

It is preferable that the target nucleotide sequence and the sequence complementary thereto contained in the siRNA be completely complementary to each other. However, in the presence of a base mutation at a position apart from the center of the siRNA (the mutation can be fall in the range of identity of at least 90% or more, preferably 95% or more), the cleavage activity by RNA interference is not completely lost, but a partial activity can remain. On the other hand, a base mutation in the center of the siRNA has a major influence to the extent that can extremely reduce the mRNA cleavage activity by RNA interference.

The siRNA may have an additional base that does not form a base pair at the 5'- and/or 3'-end. The length of the additional base is normally 5 bases or less. Although the additional base may be a DNA or an RNA, use of a DNA makes it possible to improve the stability of the siRNA. Examples of the sequences of such additional bases include, but are not limited to, the sequences ug-3', uu-3', tg-3', tt-3', ggg-3', guuu-3', gttt-3', ttttt-3', uuuuu-3' and the like.

The length of the loop portion of the hairpin loop of the shRNA is not particularly limited, as far as RNA interference can be caused; the length is normally about 5 to 25 bases. The nucleotide sequence of the loop portion is not particularly limited, as far as a loop can be formed, and the shRNA can cause RNA interference.

The polynucleotide described above can be prepared by determining the target sequence on the basis an mRNA sequence (for example, the nucleotide sequence depicted in SEQ ID NO:1) or chromosome DNA sequence of sarcopodin, and synthesizing a nucleotide sequence complementary thereto using a commercially available automated DNA/RNA synthesizer (Applied Biosystems, Beckman and the like). An siRNA can be prepared by separately synthesizing a sense strand and antisense strand using an automated DNA/RNA synthesizer, denaturing the strands in an appropriate annealing buffer solution at about 90 to about 95°C for about 1 minute, and then performing annealing at about 30 to about 70°C for about 1 to about 8 hours. A longer double-stranded polynucleotide can be prepared by synthesizing complementary oligonucleotide strands in a way such that they overlap with each other, annealing the strands, and then performing ligation with a ligase.

The inhibitor (I) of the present invention may have an expression vector capable of expressing (encoding) any polynucleotide selected from among (a) to (d) above as an active ingredient thereof. In the expression vector, the polynucleotide described above or a nucleic acid (preferably DNA) that encodes the same is operably linked to a promoter capable of exhibiting promoter activity in cells (for example, sarcoma cells) of a mammal (preferably human) which is the subject of administration.

The promoter used is not particularly limited, as far as it is capable of functioning in cells of the mammal which is the subject of administration. As the promoter, pol I promoter, pol II promoter, pol III promoter and the like can be used. Specifically, viral promoters such as the SV40-derived initial promoter and cytomegalovirus LTR, mammalian constitutive protein gene promoters such as the β-actin gene promoter, and RNA promoters such as the tRNA promoter, and the like are used.

When the expression of an siRNA is intended, it is preferable that a pol III promoter be used as the promoter. As examples of the pol III promoter, the U6 promoter, H1 promoter, tRNA promoter and the like can be mentioned.

An expression vector of the present invention preferably comprises a transcription termination signal, i.e., a terminator region, downstream of the above-described polynucleotide or nucleic acid that encodes the same. Furthermore, a selection marker gene for selection of transformant cells (a gene that confers resistance to a drug such as tetracycline, ampicillin, or kanamycin, a gene that compensates for an auxotrophic mutation and the like) can further be contained.

Although there is no limitation on the choice of expression vector used in the present invention, suitable vectors for administration to mammals such as humans include viral vectors such as retrovirus, adenovirus, and adeno-associated virus. Adenovirus, in particular, has advantages such as very high gene transfer efficiency and transferability to non-dividing cells as well. However, because the integration of transgenes into host chromosomes is extremely rare, the gene expression is transient and normally persists only for about 4 weeks. Considering the persistence of therapeutic effect, it is also preferable to use adeno-associated virus, which offers a relatively high efficiency of gene transfer, which can be transferred into non-dividing cells as well, and which can be integrated into chromosomes via an inverted terminal repeat (ITR).

Administration of the inhibitor (I) of the present invention, which comprises the polynucleotide described above or an expression vector capable of expressing the same as an active ingredient thereof, is performed by an in vivo method wherein the transfer is achieved by directly administering the vector into the body of the subject of administration. In this case, the viral vector is administered intravenously, intra-arterially, subcutaneously, intradermally, intramuscularly, intraperitoneally or the like in the form of an injection. If the production of a neutralizing antibody against the viral vector is problematic, topically injecting the vector near the affected site (sarcoma tissue) (in situ method) can lessen the adverse effect of the presence of the antibody.

The inhibitor (I) of the present invention can comprise, in addition to the above-described polynucleotide or an expression vector capable of expressing the same, an optionally chosen carrier, for example, a pharmaceutically acceptable carrier.

As examples of the pharmaceutically acceptable carrier, excipients such as sucrose and starch; binders such as cellulose and methylcellulose; disintegrants such as starch and carboxymethylcellulose; lubricants such as magnesium stearate and Aerosil; flavoring agents such as citric acid and menthol; preservatives such as sodium benzoate and sodium hydrogen sulfite; stabilizers such as citric acid and sodium citrate; suspending agents such as methylcellulose and polyvinylpyrrolidone; dispersing agents such as surfactants; diluents such as water and physiological saline; base waxes; and the like can be mentioned, which, however, are not to be construed as limiting.

To promote the introduction of a polynucleotide into cells, the inhibitor (I) of the present invention can further comprise a reagent for nucleic acid transfection. When the polynucleotide is integrated in a viral vector, particularly a retrovirus vector, retronectin, fibronectin, polybrene or the like can be used as the transfection reagent. When the polynucleotide is integrated in a plasmid vector, a cationic lipid such as lipofectin, lipofectamine, DOGS (transfectam), DOPE, DOTAP, DDAB, DHDEAB, HDEAB, polybrene, or poly(ethylenimine) (PEI) can be used.

As preparations suitable for oral administration, liquids, capsules, sachets, tablets, suspensions, emulsions and the like can be mentioned.

Preparations suitable for parenteral administration (for example, subcutaneous injection, intramuscular injection, topical injection, intraperitoneal administration and the like) include aqueous and non-aqueous isotonic sterile injectable liquids, which may contain an antioxidant, a buffer solution, a bacteriostatic agent, an isotonizing agent and the like. Aqueous and non-aqueous sterile suspensions can also be mentioned, which may contain a suspending agent, a solubilizer, a thickening agent, a stabilizer, an antiseptic and the like. These preparations can be encapsulated in containers such as ampoules and vials for unit dosage or a plurality of dosages. It is also possible to freeze-dry the active ingredient and a pharmaceutically acceptable carrier, and store the preparation in a state that may be dissolved or suspended in an appropriate sterile vehicle just before use.

The content of the polynucleotide described above in the pharmaceutical composition is, for example, about 0.1 to 100% by weight of the entire pharmaceutical composition.

Although the dosage of the inhibitor (I) of the present invention varies depending on the activity and choice of the active ingredient, seriousness of illness, recipient animal species, the recipient's drug receptivity, body weight, age, and the like, and cannot be generalized, the dosage is normally about 0.001 to about 500 mg/kg, based on the amount of active ingredient, per day for an adult.

The inhibitor (I) of the present invention is preferably safely administered to a mammal (for example, rat, mouse, guinea pig, rabbit, sheep, horse, swine, bovine, monkey, human) in a way such that the above-described polynucleotide or expression vector capable of expressing the same, which is the active ingredient thereof, will be delivered to a sarcoma tissue (sarcoma cells).

Because the above-described polynucleotide possesses an activity to inhibit the motility or invasive potential of sarcoma cells, metastasis of a sarcoma (particularly spindle cell sarcoma) can be inhibited to improve the prognosis for survival by administering the inhibitor (I) of the present invention to a patient with a sarcoma (particularly spindle cell sarcoma) or a patient after treatment of a sarcoma at a risk of distant metastasis of the sarcoma and the like.

### 5. Screening method (I) for substance capable of inhibiting sarcoma metastasis and the like

The present invention also provides a method of screening for a substance capable of inhibiting the metastasis of a sarcoma or the motility or invasive potential of a sarcoma cell, comprising evaluating whether or not the test substance suppresses the expression of sarcopodin, and a substance that can be obtained by the method. In the screening method (I) of the present invention, a substance that suppresses the expression of sarcopodin is selected as a substance capable of inhibiting the metastasis of a sarcoma (particularly spindle cell sarcoma) or the motility or invasive potential of sarcoma cells (particularly spindle cell sarcoma cells).

The test substance subjected to the screening method (I) of the present invention may be any commonly known compound or a novel compound; examples include nucleic acids, sugars, lipids, proteins, peptides, organic low molecular compounds, compound libraries prepared using combinatorial chemistry technology, random peptide libraries, or naturally occurring ingredients derived from microorganisms, animals, plants, marine organisms and the like, and the like.

The screening method (I) of the present invention comprises the following steps of:
(I) bringing into contact with each other a test substance and a cell that permits a measurement of the expression of any polypeptide or polynucleotide selected from among (1) to (5) below;
   (1) a polypeptide comprising the amino acid sequence depicted in SEQ ID NO:2,
   (2) a polypeptide comprising an amino acid sequence having an identity of 90% or more to the amino acid sequence depicted in SEQ ID NO:2, and possessing an activity to enhance the motility or invasive potential of a cell when expressed in the cell,
   (3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell,
   (4) a polynucleotide comprising a coding region in the nucleotide sequence depicted in SEQ ID NO:1, and
   (5) a polynucleotide comprising a nucleotide sequence that encodes any polypeptide selected from among (1) to (3) above;
(II) measuring the amount of any polypeptide or polynucleotide selected from among (1) to (5) above expressed in the cell contacted with the test substance, and comparing this amount expressed with the amount of the above-described polypeptide or polynucleotide expressed in a control cell not contacted with the test substance; and
(III) selecting a test substance that suppresses any polypeptide or polynucleotide selected from among (1) to (5) below as a substance capable of inhibiting the metastasis of a sarcoma or the motility or invasive potential of a sarcoma cell on the basis of the results of the comparison in (II) above.

The expression of any polypeptide or polynucleotide selected from among (1) to (5) accordingly means the expression of a polypeptide or polynucleotide (preferably mRNA) of sarcopodin. The definitions for the polypeptides or polynucleotides (1) to (5) are the same as those described in the (1. Method of determining the risk of distant metastasis of tumor or the prognosis for survival of tumor patient) section.

"A cell that permits a measurement of the expression" refers to a cell that permits a direct or indirect evaluation of the expression level of an mRNA or protein which is the subject of measurement. As a cell that permits a direct evaluation of the expression level of an mRNA or protein which is the subject of measurement, a cell capable of expressing the subject of measurement in nature can be mentioned; as a cell that permits an indirect evaluation of the expression level of an mRNA or protein which is the subject of measurement, a cell that permits a reporter assay for a transcriptional regulatory region of the subject of measurement can be mentioned.

The cell capable of expressing the subject of measurement, that is, sarcopodin, in nature is not particularly limited, as far as it potentially expresses the polypeptides or polynucleotides (1) to (5) above; as the cell, a primary culture cell, a cell line induced from the primary culture cell and the like can be used. As examples of cells capable of expressing sarcopodin in nature, sarcoma cells and the like can be mentioned.

The cell that permits a reporter assay for a transcriptional regulatory region of the subject of measurement, i.e., sarcopodin, is a cell comprising a transcriptional regulatory region (for example, a DNA consisting of the base sequence of about 2 kbp upstream of the transcription initiation point) of the sarcopodin gene and a reporter gene (for example, GFP gene) operably linked to the region. A sarcoma cell is preferred as the cell which is the subject of measurement because the cell expresses a physiological transcriptional regulatory factor for sarcopodin and is thought to be more appropriate for an evaluation of the regulation of the expression of sarcopodin.

The contact of a test substance and a cell that permits a measurement of the expression of sarcopodin is performed in a culture medium. A culture medium is chosen as appropriate according to the cell that permits a measurement of the expression of sarcopodin; examples include minimal essential medium (MEM), Dulbecco's modified essential medium (DMEM), and the like containing about 5 to 20% fetal bovine serum. Cultivation conditions are also determined as appropriate; for example, the pH of the medium is about 6 to about 8, cultivation temperature is normally about 30 to about 40°C, and cultivation time is about 12 to about 72 hours.

When a cell capable of expressing sarcopodin is used, a measurement of the amount of sarcopodin (polypeptides or polynucleotides (1) to (5)) expressed can be performed according to the method described in the (1. Method of determining the risk of distant metastasis of tumor or the prognosis for survival of tumor patient) section. When a cell containing a reporter gene is used, the amount expressed is measured on the basis of the signal intensity of the reporter gene.

The comparison of the amounts expressed can be made preferably on the basis of the presence or absence of a significant difference. Although the amount of sarcopodin expressed in the control cell not contacted with the test substance may be an amount expressed measured before or simultaneously with the measurement of the amount of sarcopodin expressed in the cell contacted with the test substance, it is preferable, from the viewpoint of experimental accuracy and reproducibility, that the former amount expressed be a simultaneously measured amount expressed.

Then, a substance that suppresses the amount of sarcopodin expressed, obtained as a result of the comparison, is selected as a substance capable of inhibiting the metastasis of a sarcoma or the motility or invasive potential of sarcoma cells.

A compound obtained by the screening method (I) of the present invention is useful as a candidate substance for the development of a novel inhibitory agent for the metastasis of a sarcoma.

### 6. Inhibitor of sarcoma metastasis or sarcoma cell motility

As shown in Examples below, by introducing the shorter form of sarcopodin into a sarcoma cell expressing the longer form thereof, the motility of this sarcoma cell can be inhibited. Therefore, the shorter-form polypeptide of sarcopodin and an expression vector capable of expressing the same are useful as agents for inhibiting sarcoma metastasis.
Accordingly, the present invention provides an agent for inhibiting the metastasis of a sarcoma or the motility of a sarcoma cell, containing any polypeptide selected from among (1) to (3) below or an expression vector capable of expressing the polypeptide (inhibitor (II) of the present invention):
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing the amino acid sequence depicted in SEQ ID NO:2 when expressed in the sarcoma cell.

The shorter-form sarcopodin polypeptide used in the inhibitor (II) of the present invention possesses an activity to inhibit the motility of a sarcoma cell expressing a polypeptide comprising the amino acid sequence depicted in SEQ ID NO:2 when expressed in the sarcoma cell. The activity can be the inhibition of the cell motility enhancing activity of a polypeptide comprising the amino acid sequence depicted in SEQ ID NO:2 (longer-form sarcopodin). The presence or absence of such an activity of shorter-form sarcopodin can be determined by introducing an expression vector capable of expressing a desired polypeptide into a sarcoma cell expressing longer-form sarcopodin, and comparing the motility of the cell obtained with the motility of a control sarcoma cell.

The amino acid sequence contained in the polypeptide (2) above is an amino acid sequence having an identity of 90% or more, preferably 95% or more, more preferably 98% or more, still more preferably 99% or more, to the amino acid sequence depicted in SEQ ID NO:4. The definition for "identity" is the same as that described in the (1. Method of determining the risk of distant metastasis of tumor or the prognosis for survival of tumor patient) section.

The amino acid sequence contained in the polypeptide (3) above is an amino acid sequence resulting from deletion, substitution, insertion or addition of one or a plurality of amino acids in the amino acid sequence depicted in SEQ ID NO:4, for example, (1) an amino acid sequence resulting from deletion of one or a plurality of (preferably 1 to 30, more preferably 1 to 10, still more preferably one to several (2 to 5)) amino acids in the amino acid sequence depicted in SEQ ID NO:4, (2) an amino acid sequence resulting from addition of one or a plurality of (preferably 1 to 30, more preferably 1 to 10, still more preferably one to several (2 to 5)) amino acids in the amino acid sequence depicted in SEQ ID NO:4, (3) an amino acid sequence resulting from insertion of one or a plurality of (preferably 1 to 30, more preferably 1 to 10, still more preferably one to several (2 to 5)) amino acids in the amino acid sequence depicted in SEQ ID NO:4, (4) an amino acid sequence resulting from substitution of one or a plurality of (preferably 1 to 30, more preferably 1 to 10, still more preferably one to several (2 to 5)) amino acids in the amino acid sequence depicted in SEQ ID NO:4 by other amino acids, or (5) an amino acid sequence being a combination of the mutations (1) to (4) above (in this case, the total number of mutated amino acids is preferably 1 to 30, more preferably 1 to 10, still more preferably one to several (2 to 5)).

In a preferred embodiment, the polypeptides (1) to (3) above are natural shorter-form human sarcopodin polypeptides. "Natural" refers to the existence of an amino acid sequence that constitutes a polypeptide in nature. Natural shorter-form human sarcopodin amino acid sequences include, but are not limited to, the amino acid sequence depicted in SEQ ID NO:4. It is known that a polymorphism (individual differences) usually exists in gene-constituting nucleotide sequences. Polypeptides having an amino acid sequence different from the amino acid sequence depicted in SEQ ID NO:4, resulting from such polymorphism of the sarcopodin gene, are also included in natural shorter-form human sarcopodin polypeptides, as far as they possess an activity to suppress the motility of a sarcoma cell when expressed in the sarcoma cell.

The polypeptides (1) to (3) above may comprise a tag for facilitating protein detection, purification and the like, and an additional sequence for facilitating protein introduction into a cell, such as a Protein Transduction Domain (PTD). More specifically, as the tag, a Flag tag, histidine tag, c-Myc tag, HA tag, AU1 tag, GST tag, MBP tag, fluorescent protein tag (for example, GFP, YFP, RFP, CFP, BFP and the like), immunoglobulin Fc tag and the like can be mentioned. As the PTD, transcellular domains such as ANTENNAPEDIA, HIV/TAT, and HSV/VP-22, 7 to 11 polyarginines and the like can be mentioned. Although the position of the additional sequence is not particularly limited, as far as the activity of the polypeptides (1) to (3) above to inhibit the motility of sarcoma cells is not affected, the position is preferably a terminal (N-terminal or C-terminal) of the polypeptide.

An expression vector capable of expressing any polypeptide selected from among (1) to (3) above can also be an active ingredient for the inhibitor (II) of the present invention. In the expression vector, usually, a polynucleotide (preferably DNA) comprising a nucleotide sequence that encodes the polypeptide described above (for example, the nucleotide sequence depicted in SEQ ID NO:3) is operably linked to a promoter capable of exhibiting promoter activity in cells (for example, sarcoma cells) of a mammal (preferably human) which is the subject of administration.

As the promoter used in the present invention, those mentioned in the (4. Agent for inhibiting the metastasis of sarcoma or the motility or invasive potential of sarcoma cells) section can be mentioned.

The expression vector preferably comprises a transcription termination signal, i.e., a terminator region, downstream of a polynucleotide comprising a nucleotide sequence that encodes the above-described polypeptide. Furthermore, a selection marker gene for selection of transformant cells (a gene that confers resistance to a drug such as tetracycline, ampicillin, or kanamycin, a gene that compensates for an auxotrophic mutation, and the like) can further be contained.

As the vector used in the present invention, those mentioned in the (4. Agent for inhibiting the metastasis of sarcoma or the motility or invasive potential of sarcoma cells) section can be mentioned.

The inhibitor (II) of the present invention can comprise, in addition to the above-described polypeptide or an expression vector capable of expressing the same, an optionally chosen carrier, for example, a pharmaceutically acceptable carrier, and can be prepared as a medicine preparation in the same manner as with the inhibitor (I) of the present invention.

The content of the above-described polypeptide or an expression vector capable of expressing the same in the pharmaceutical composition is, for example, about 0.1 to 100% by weight of the entire pharmaceutical composition.

Although the dosage of the inhibitor (II) of the present invention varies depending on the activity and choice of the active ingredient, seriousness of illness, recipient animal species, the recipient's drug receptivity, body weight, age, and the like, and cannot be generalized, the dosage is normally about 0.001 to about 500 mg/kg, based on the amount of active ingredient, per day for an adult.

The inhibitor (II) of the present invention is preferably administered to a mammal (for example, rat, mouse, guinea pig, rabbit, sheep, horse, swine, bovine, monkey, human) in a way such that the above-described polypeptide or an expression vector capable of expressing the same, which is an active ingredient thereof, is delivered to a sarcoma tissue (sarcoma cells).

Since the above-described polypeptide possesses an activity to inhibit the motility or invasive potential of a sarcoma cell, metastasis of sarcomas (particularly spindle cell sarcoma) can be inhibited to improve the prognosis for survival by administering the inhibitor (II) of the present invention to a patient with a sarcoma (particularly spindle cell sarcoma) or a patient after treatment of a sarcoma at a risk of distant metastasis of the sarcoma, and the like.

### 7. Screening method (II) for substance capable of inhibiting sarcoma metastasis and the like

As stated above, by introducing the shorter-form of sarcopodin into a sarcoma cell expressing the longer form thereof, the motility of this sarcoma cell can be inhibited. This finding shows that by inhibiting a function of longer-form sarcopodin using an appropriately designed deletion mutant sarcopodin (partial peptide), it is possible to inhibit the motility of a sarcoma cell and hence inhibit the metastasis of a sarcoma. As shown in Examples, longer-form sarcopodin binds to actin and Src. Therefore, the deletion mutant is possibly one capable of inhibiting this binding, but this mode of inhibition is not to be construed as limiting.

On the basis of these findings, the present invention provides a method of screening for a polypeptide capable of inhibiting the metastasis of a sarcoma or the motility of sarcoma cells, comprising the following steps of:
(I) expressing a test polypeptide comprising at least three consecutive amino acids contained in the amino acid sequence depicted in SEQ ID NO:2 in a sarcoma cell expressing a polypeptide comprising the amino acid sequence depicted in SEQ ID NO:2;
(II) measuring the motility of the cell in which the test polypeptide is expressed, and comparing this motility with the motility of a control sarcoma cell in which the test polypeptide is not expressed;
(III) selecting a polypeptide that has suppressed the motility of the sarcoma cell as a polypeptide capable of inhibiting the metastasis of a sarcoma or the motility of sarcoma cells on the basis of the results of the comparison in (II) above , and a polypeptide that can be obtained by the method.

The test polypeptide subjected to the screening method (II) of the present invention comprises a partial sequence consisting of at least three consecutive amino acids contained in the amino acid sequence depicted in SEQ ID NO:2. The length of the partial sequence is not particularly limited; however, to achieve a sufficient inhibitory activity, the length is preferably 5 amino acids or more, more preferably 10 amino acids or more.

Because the motility of a sarcoma cell can be inhibited by introduction of shorter-form sarcopodin, a polypeptide that potently inhibits the motility of a sarcoma cell can be obtained at a higher probability by designing a test polypeptide on the basis of this shorter-form sarcopodin. Therefore, in a preferred embodiment, the test polypeptide subjected to the screening method (II) of the present invention comprises a partial sequence consisting of at least three consecutive amino acids contained in the amino acid sequence depicted in SEQ ID NO:4.

The expression of a test polypeptide in a sarcoma cell can be achieved by introducing an expression vector capable of expressing the test polypeptide into the sarcoma cell. In the expression vector, a polynucleotide comprising a nucleotide sequence that encodes the test polypeptide is operably linked to a promoter capable of exhibiting promoter activity in the sarcoma cell.

As the promoter used, those mentioned in the (4. Agent for inhibiting the metastasis of sarcoma or the motility or invasive potential of sarcoma cells) section can be mentioned.

The expression vector preferably comprises a transcription termination signal, i.e., a terminator region, downstream of a polynucleotide comprising a nucleotide sequence that encodes the test polypeptide. Furthermore, a selection marker gene for selection of transformant cells (a gene that confers resistance to a drug such as tetracycline, ampicillin, or kanamycin, a gene that compensates for an auxotrophic mutation, and the like) can further be contained.

As the vector used in the present invention, those mentioned in the (4. Agent for inhibiting the metastasis of sarcoma or the motility or invasive potential of sarcoma cells) section can be mentioned.

Introduction of an expression vector into a sarcoma cell can be performed by an ordinary method of transfection, such as the calcium phosphate method, DEAE dextran method, electroporation, or lipofection. When a virus is used as the vector, the genome of the virus may be introduced into the cell by one of the ordinary method of transfection described above, and the genome of the virus can also be introduced into the cell by infecting the cell with a virion.

The sarcoma cell used in a method of the present invention is normally derived from a mammal, preferably derived from a human. The sarcoma cell is expressing a polypeptide comprising the amino acid sequence depicted in SEQ ID NO:2 (longer-form human sarcopodin) naturally or forcibly. Forced expression of the polypeptide can be achieved by introducing an expression vector capable of expressing the polypeptide, as described above.

The motility of a cell in which the test polypeptide is expressed can be measured according to, for example, the gold colloid method, Boyden chamber assay and the like.

Then, the motility of the cell in which the test polypeptide is expressed is compared with the motility of a control sarcoma cell in which the test polypeptide is not expressed.

The comparison of the motilities can be made preferably on the basis of the presence or absence of a significant difference. Although the motility of the control cell in which the test polypeptide is not expressed may be measured before or simultaneously with the measurement of the motility of the cell in which the test polypeptide is expressed, it is preferable, from the viewpoint of experimental accuracy and reproducibility, that the former motility be a simultaneously measured value.

Then, a polypeptide that has suppressed the motility of the sarcoma cell, obtained as a result of the comparison, is selected as a polypeptide capable of inhibiting the metastasis of a sarcoma or the motility of sarcoma cells.

A polypeptide obtained by the screening method (II) of the present invention is useful as a candidate substance for the development of a novel inhibitory agent for sarcoma metastasis.

The present invention is hereinafter described in more detail by means of the following Examples, to which, however, the present invention is not limited in any way.

### Examples

### Example 1: Expression of sarcopodin in spindle cell sarcoma and functional analysis thereof Identification of sarcopodin gene

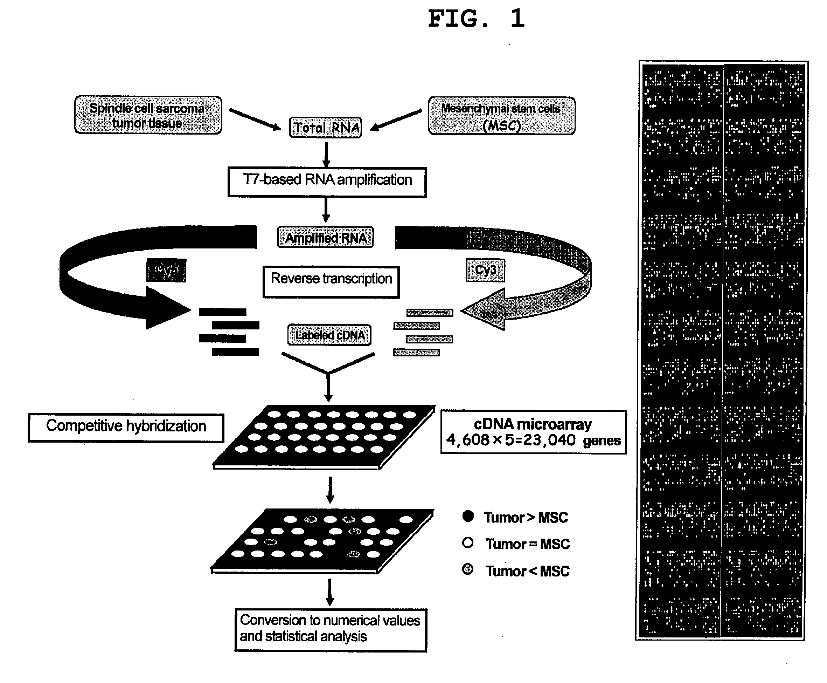

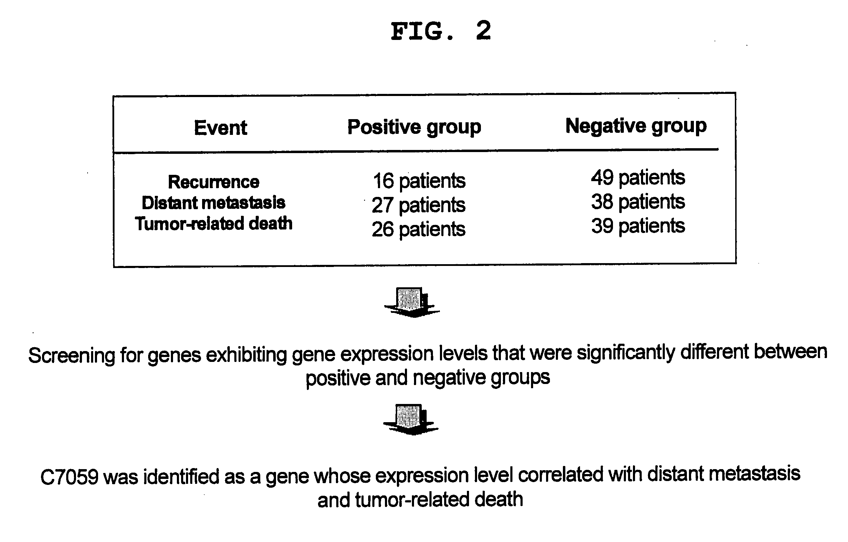

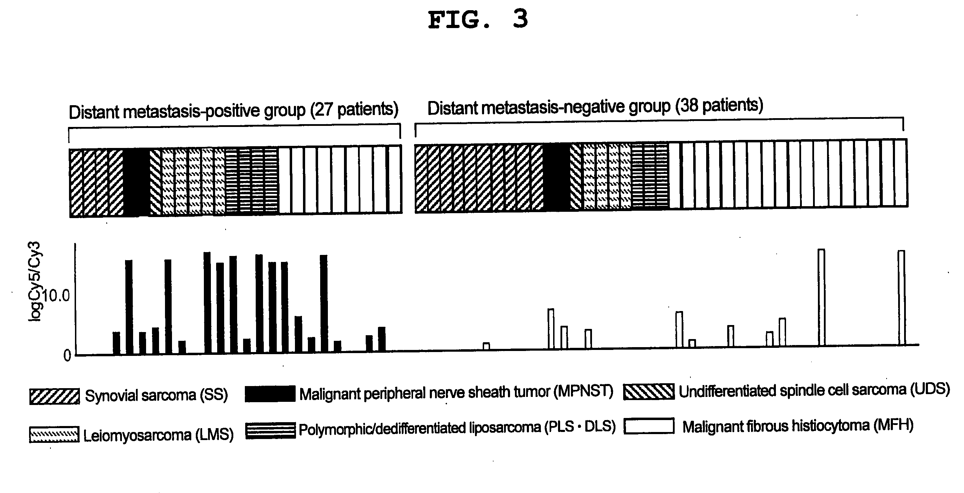

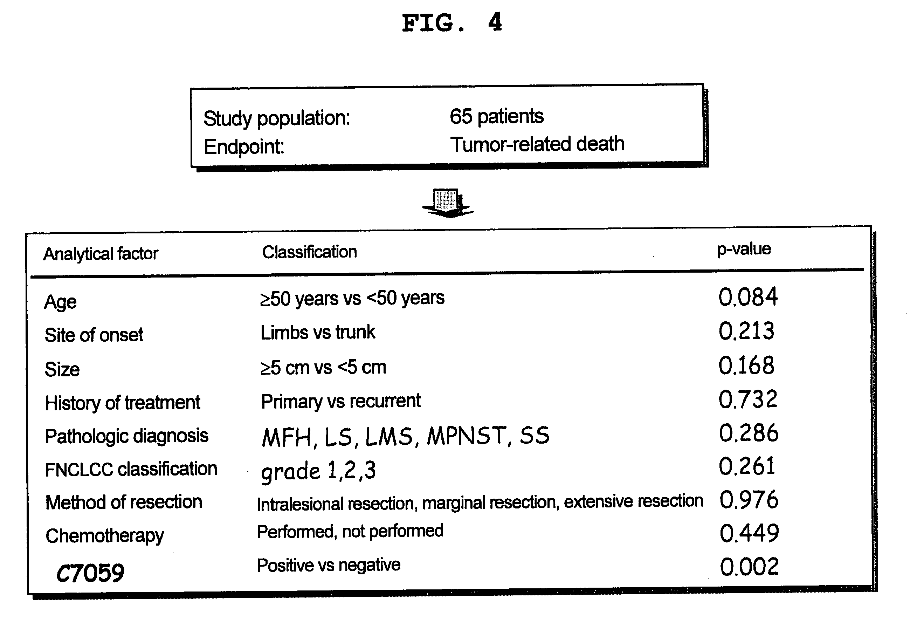

In the Examples below, the longer form of sarcopodin is referred to as C7059, and the shorter form of sarcopodin as ctC7059.
cDNAs were prepared from RNAs extracted from spindle cell sarcoma tissues of 65 patients, and a comprehensive gene expression analysis was performed using a cDNA microarray on about 23000 kinds of genes prepared at the Institute of Medical Science in the University of Tokyo (Fig. 1). As a reference control, a cDNA prepared from an RNA extracted from mesenchymal stem cells (hereinafter MSCs) was used. The 65 patients were divided into two groups according to the presence or absence of onset of distant metastasis or tumor-related death, and they were examined for genes exhibiting a statistically significant difference in the expression level between the two groups. As a result, C7059 was identified as a gene exhibiting the strongest correlation with both the onset of distant metastasis and tumor-related death (Fig. 2).
On the basis of the pathologic findings, spindle cell sarcoma patients were classified into some groups; irrespective of the pathologic classification, the expression level of C7059 was high in patients positive for distant metastasis (Fig. 3).
For factors other than the amount of gene expressed as well, the correlations with tumor-related death in spindle cell sarcoma were analyzed. However, of the factors analyzed, only the C7059 expression level exhibited a significant correlation with tumor-related death (Fig. 4).
These results suggested that by measuring the expression level of C7059 in spindle cell sarcoma tissue, the risk of onset of distant metastasis or the prognosis for survival of the patient could be determined.

### Analysis of information on C7059

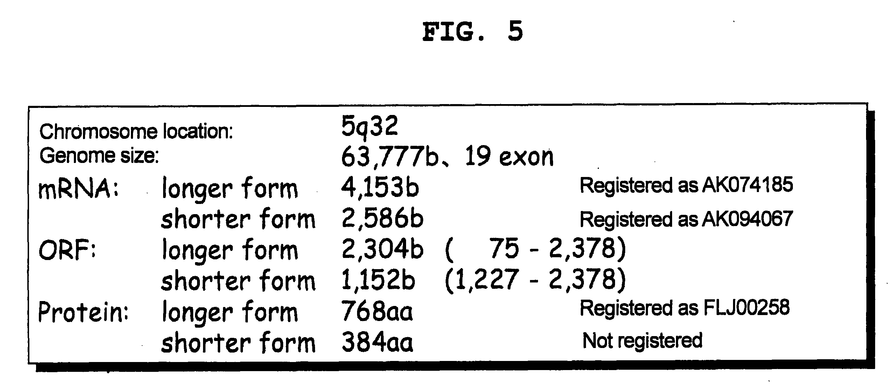

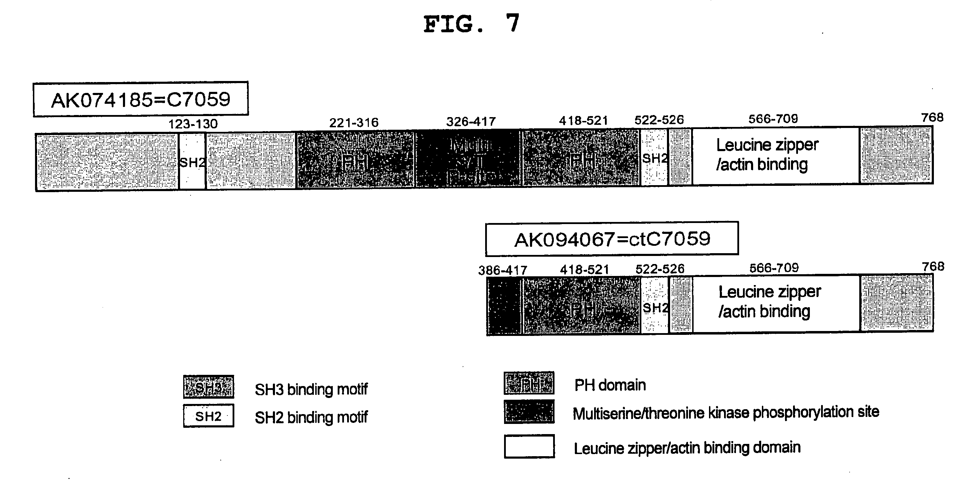

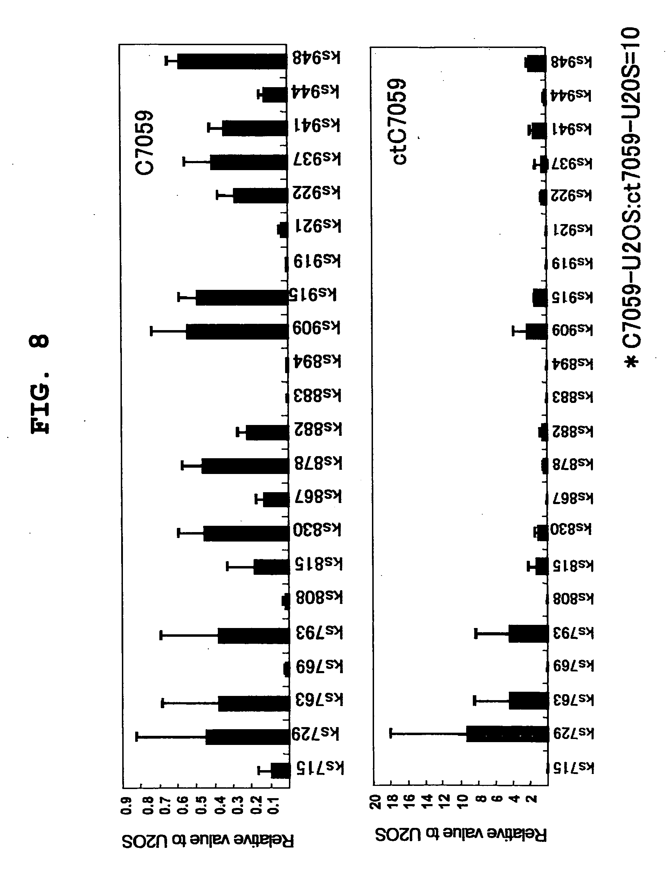

C7059 was identified as a functionally unknown gene registered with GenBank as AK074185 (SEQ ID NO:1). C7059 is a gene present in the long arm region of chromosome 5 (5q32), consisting of 19 exons; the cDNA and ORF thereof were found to have lengths of 4153bp and 2304bp, respectively, and encode a protein configured with 768 amino acids (accession number: FLJ00258) (SEQ ID NO:2) (Fig. 5). This gene also have a transcription initiation point within the 9th intron, the shorter form of C7059 (referred to as ctC7059), having the same structure as the C-terminal of C7059, was found to be registered as AK094067 (SEQ ID NO:3) (Figs. 6 and 7).
A portion of a DNA that encodes C7059 was amplified by PCR using the following primers, and the portion was used as a probe for cDNA microarray.
Forward: tggtggtgcagtgtaaagagacaaga (SEQ ID NO:1 nucleotide numbers 2786 to 2811: SEQ ID NO:5)
Reverse: gtttagcacccatgtgacagaa (SEQ ID NO:1 nucleotide numbers 3355 to 3334: SEQ ID NO:6)
This probe hybridizes to a common region shared by C7059 and ctC7059. Hence, the sum of the amounts of C7059 and ctC7059 expressed is detected.
It was also found by quantitative RT-PCR that the expression of ctC7059 in spindle cell sarcoma was lower than the expression of C7059 (Fig. 8). From these findings, it was estimated that the amount of C7059 expressed mainly correlates with the onset of distant metastasis and tumor-related death.
The nucleotide sequences of the primers used in the above-described RT-PCR are as follows:
Primers for C7059 amplification
Forward: atcggcagccacatctgaggttg (SEQ ID NO:1 nucleotide numbers 841 to 863: SEQ ID NO:7)
Reverse: ttgtcacccacacccacgct (SEQ ID NO:1 nucleotide numbers 1150 to 1131: SEQ ID NO:8)
Primers for ctC7059 amplification
Forward: gtatggtatgaggcacaggatag (SEQ ID NO:3 nucleotide numbers 30 to 52: SEQ ID NO:9)
Reverse: gcgttcacatgggttcgcag (SEQ ID NO:1 nucleotide numbers 1453 to 1434: SEQ ID NO:10)
This finding suggested that the risk of the onset of distant metastasis of spindle cell sarcoma or the prognosis for survival of the patient could be determined by specifically detecting C7059.

### Verification of microarray data

Probes for quantitative RT-PCR were generated from the genome sequence of C7059, and the expression of C7059 in the spindle cell sarcoma tissue used in the microarray was quantitatively analyzed by the TaqMan method.
The nucleotide sequences of the primers and probe used in the TaqMan method are as follows:
Forward: aacaagcctccccctgaggact (SEQ ID NO:1 nucleotide numbers 429 to 450: SEQ ID NO:11)
Reverse: gttgcagggtagctgctgtc (SEQ ID NO:1 nucleotide numbers 580 to 561: SEQ ID NO:12)
TaqMan probe: ggcccttcctctgggacccggc (SEQ ID NO:1 nucleotide numbers 461 to 482: SEQ ID NO:13)
Because these primers are specific for C7059 and do not detect ctC7059, the amount of only C7059 expressed can be measured quantitatively.
As a result, the results of the quantitative RT-PCR generally agreed with the microarray data (Figs. 8A and B); with the expression level of reference control mesenchymal stem cells taken as 1, the patients were divided into two groups: a group having an expression level of 10 or more and a group having an expression level of less than 10; the incidence of distant metastasis were compared; in the group with an expression level of 10 or more, distant metastasis occurred at a higher rate with a statistically significant difference (Figs. 9A and B). Furthermore, a similar analysis was performed using the results from 10 newly enrolled patients; as expected, in patients with high expression of C7059, distant metastasis occurred at a higher frequency (Figs. 9A and C).

### Preparation of specific antibody

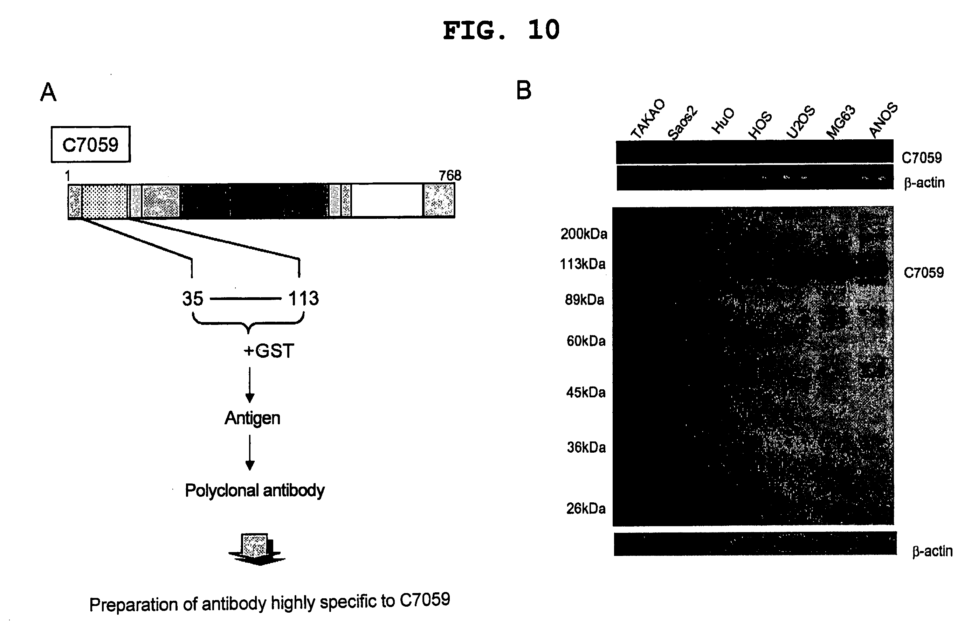

Domestic rabbits were immunized with a fusion protein of an amino-terminal region of C7059 (the region shown by amino acid numbers 35 to 113 in SEQ ID NO:2) and GST as an antigen to generate a C7059-specific polyclonal antibody (Fig. 10A). By Western blot using the antibody obtained, C7059 was found to be an about 110kD protein present mainly in the cytoplasm (Fig. 10B).

### Immunohistochemical staining

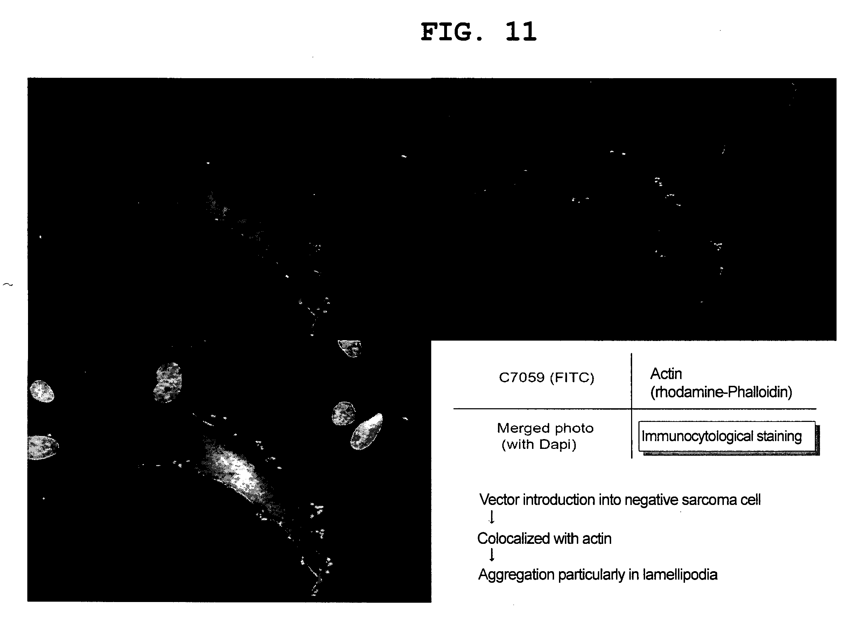

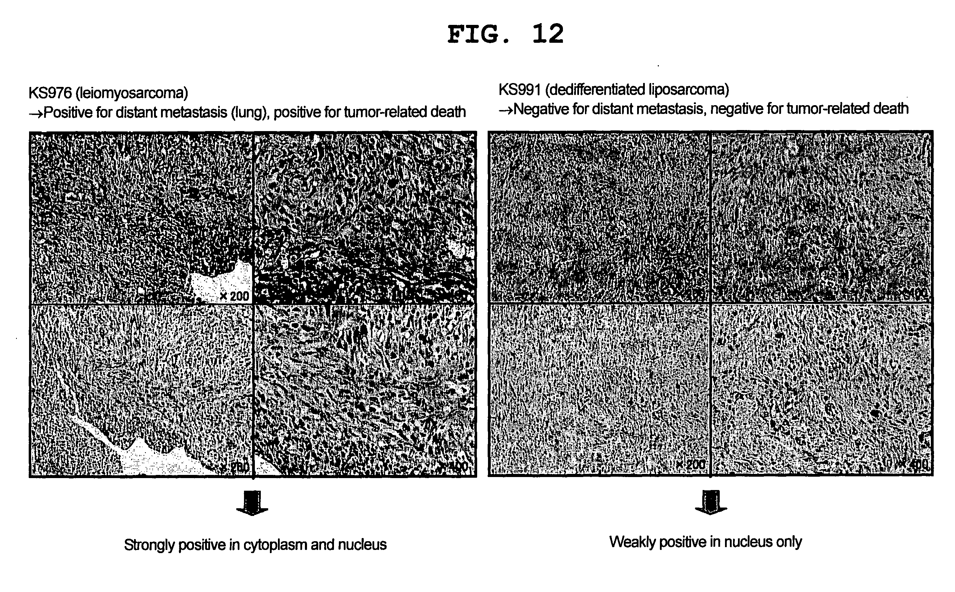

The C7059 expression vector described below was introduced into C7059-negative sarcoma cells (SaOS2). The cells were stained using the anti-C7059 antibody described above. As a result, C7059 was found to bind with actin and aggregate particularly in the lamellipodia (Fig. 11).
Formalin-fixed tumor tissues collected from spindle cell sarcoma patients were stained using the anti-C7059 antibody described above. As a result, C7059 stainability was observed mainly in the cytoplasm, and partially in the nucleus. Agreeing with the analytical results at the RNA level, the sarcoma tissue from the patient positive for distant metastasis and positive for tumor-related death was strongly positive for C7059, and the sarcoma tissue from the patient negative for distant metastasis and negative for tumor-related death was weakly positive for C7059 (Fig. 12). This result showed that the risk of onset of distant metastasis in spindle cell sarcoma and the prognosis for survival of the patient could also be determined by immunohistochemical staining using a tissue section.
The sarcoma tissue from the patient positive for distant metastasis and positive for tumor-related death exhibited strong C7059 expression in the cytoplasm and nucleus, whereas the sarcoma tissue from the patient negative for distant metastasis and negative for tumor-related death exhibited weak C7059 expression in the nucleus only (Fig. 12). This result suggested that the intracellular localization of the C7059 protein might vary depending on the malignancy of sarcoma cells.

### Effects of induction or inhibition of C7059 expression on cell motility and invasive potential

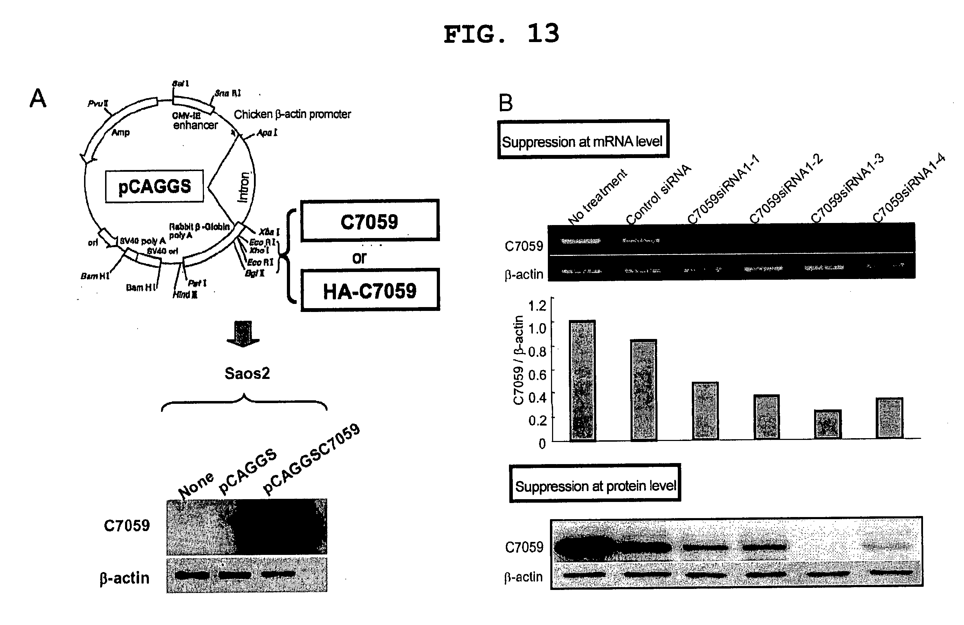

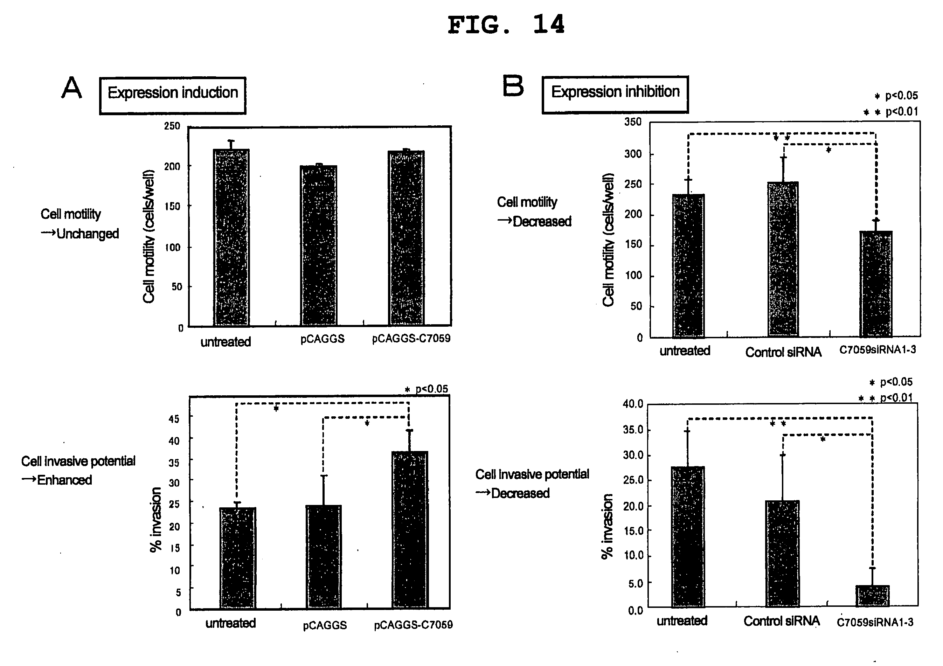

By inserting a DNA that encodes C7059 into a multicloning site of pCAGGS, a vector capable of transiently strongly expressing the C7059 protein was prepared (Fig. 13). The C7059 expression vector was introduced into a C7059-negative cultured sarcoma cell (SaOS2), and the motility and invasive potential of the cell were evaluated.
The motility and invasive potential of the cell were evaluated by Boyden chamber assay. 2.5×10⁴ of the cells were seeded to a 24-well plate having a PET membrane with 8-micron pores on the bottom thereof; 22 hours later, the cells that had passed the membrane were counted to obtain an index of cell motility. Next, using a porous PET membrane coated with a synthetic basal membrane tissue, the same experiment was performed; the cells that destroyed the basal membrane tissue and invaded were counted. Using the quota obtained by dividing the number of invasive cells by the number of migrating cells, the cell invasive potential was evaluated.
As a result, with the introduction of the C7059 expression vector, the sarcoma cell motility did not change, but the invasive potential was significantly enhanced (Fig. 14A). This result suggested that an elevation of C7059 expression in spindle cell sarcoma might cause an enhancement of the invasive potential, which in turn can result in an increased incidence of distant metastasis and a worse prognosis for survival of the patient.

The following 4 kinds of siRNAs against C7059 were designed:
C7059 siRNA1-1: cagggugaacggcgagcuuaa (SEQ ID NO:1 nucleotide numbers 581 to 601: SEQ ID NO:14)
C7059 siRNAl-2: cagcaucaucuacgugcccaa (SEQ ID NO:1 nucleotide numbers 878 to 898: SEQ ID NO:15)
C7059 siRNAl-3: aucccucuuugaagaauuuga (SEQ ID NO:1 nucleotide numbers 284 to 304: SEQ ID NO:16)
C7059 siRNAl-4: aaaggaggucuccuaccugua (SEQ ID NO:1 nucleotide numbers 221 to 241: SEQ ID NO:17)
Double-stranded RNAs were prepared from these siRNAs and introduced into C7059-positive cultured sarcoma cells (U2OS); suppression of C7059 expression at the mRNA level and protein level was confirmed (Fig. 13B). Of these RNAs, that having the most potent suppressive effect, C7059 siRNAl-3, was used in the following assay.
C7059 siRNA1-3 was introduced into a C7059-positive cultured sarcoma cell, and the motility and invasive potential of the cell were evaluated.
As a result, it was found that by inhibition of C7059 expression by the siRNA, the motility and invasive potential of the sarcoma cell were remarkably inhibited (Fig. 14B). This result suggested that by suppressing C7059 expression in sarcoma cells, the metastasis of a sarcoma might be inhibited to improve the prognosis for survival of the patient.

### Establishment of immortalized MSCs that stably express C7059 using lentivirus vector

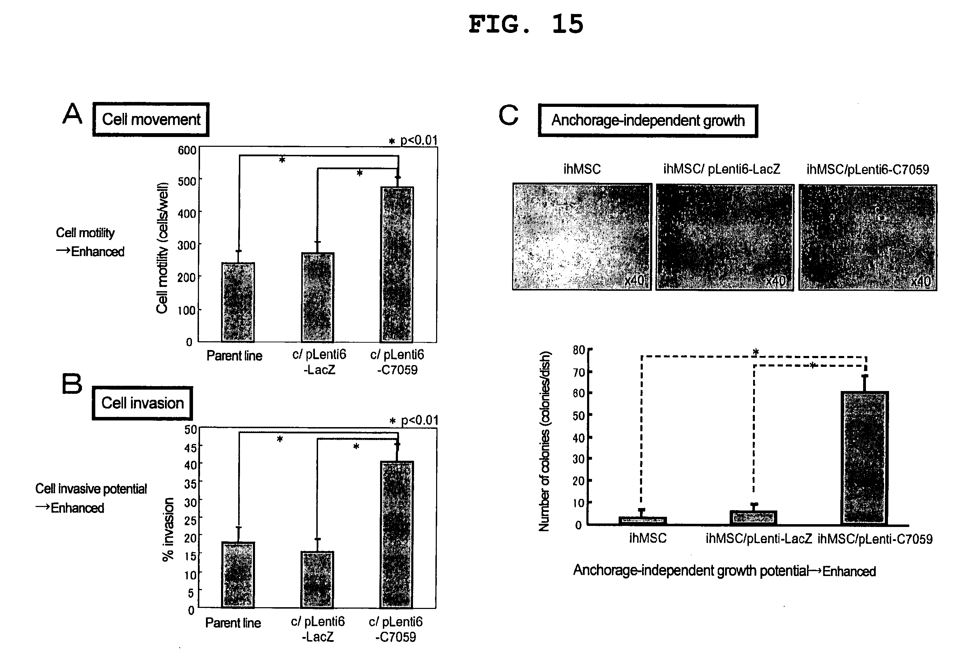

A lentivirus vector capable of strongly expressing the C7059 protein was constructed.
The region of nucleotide numbers 75 to 2381 in SEQ ID NO:1 was amplified by PCR and inserted into the cloning vector pENTR/D/TOPO by TOPO cloning, whereby pENTR/C7059 was prepared.
Subsequently, pENTR/C7059 and pLenti6/V5-DEST were LR-recombined, and pLenti/C7059 was prepared by double selection with ampicillin and blasticidin.
As described in BBRC, vol.353, p.60-66 (2007) and J Gene Med, vol.6, p.833-845 (2004), the Bmi1 gene and the human TERT gene were introduced into human MSCs (mesenchymal stem cells) purchased from BIOWHITTAKER Co., using a retrovirus, and chemical selection (G418 100 µg/ml, or hygromycin B 50 µg/ml) was performed, whereby an immortalized parent MSC line was acquired.
The C7059 expression lentivirus vector was introduced into the immortalized MSCs to establish an immortalized MSC line that expresses C7059 stably, and the motility, invasive potential and anchorage-independent growth of the cells were evaluated.
The anchorage-independent growth was evaluated by the method described below. A double agar medium comprising 0.7% of agar in the lower layer and 0.35% of agar in the upper layer was prepared using a 60mm dish; 1×10⁴ cells were seeded to the upper layer. After cultivation for 4 weeks, the colonies formed were stained and counted.
As a result, with the introduction of the C7059 expression vector, the motility, invasive potential and anchorage-independent growth potential of the MSCs were significantly enhanced (Fig. 15). This result suggested that induction of the expression of C7059 might transform immortalized MSCs cancerous.

### Binding of C7059 and Src

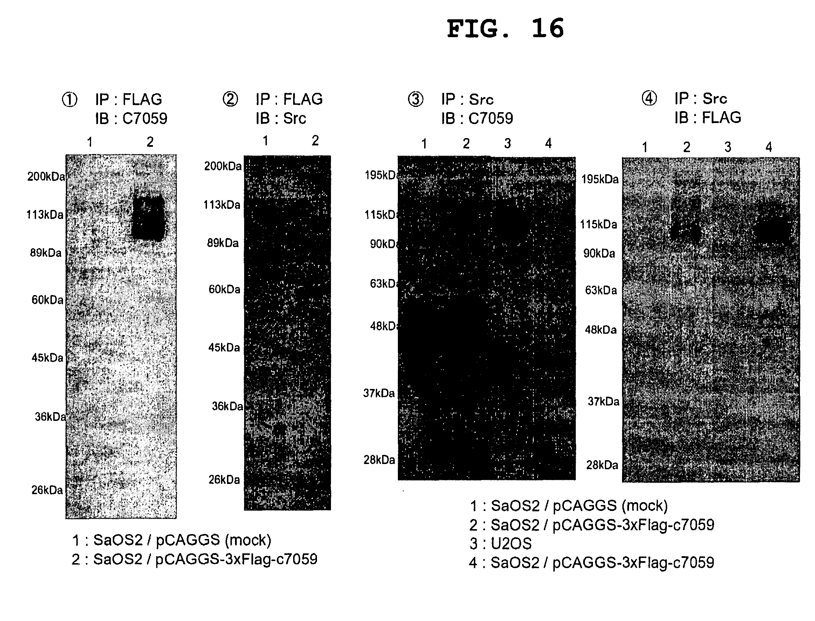

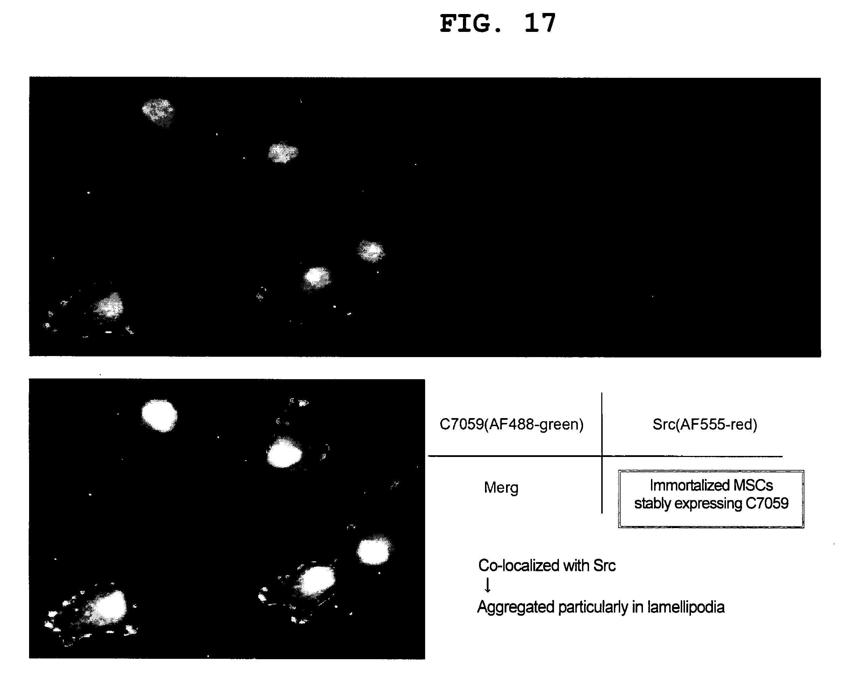

By inserting a DNA that encodes a polypeptide comprising C7059 and three Flag tags linked to the N-terminus thereof (3xFlag-c7059) into a multicloning site of pCAGGS, a vector capable of transiently strongly expressing the 3xFlag-c7059 protein was prepared. pCAGGS-3xFlag-c7059 was introduced into C7059-negative sarcoma cells (SaOS2), and the cells were analyzed for the presence or absence of an interaction between C7059 and Src by immunoprecipitation.
When cells were immunoprecipitated with anti-Flag antibody and blotted with anti-Src antibody, a specific band was identified only in the cells transfected with pCAGGS-3xFlag-c7059 (Fig. 16(2)). Even when cells were immunoprecipitated with the anti-Src antibody and blotted with the anti-C7059 antibody or anti-Flag antibody, a specific band was found only in the cells transfected with pCAGGS-3xFlag-c7059 (Figs. 16(3) and (4)).
These results demonstrated that C7059 has the capability of binding to Src. Immunocytological staining also showed that C7059 was co-localized with Src and aggregated intensely particularly in the lamellipodia (Fig. 17).

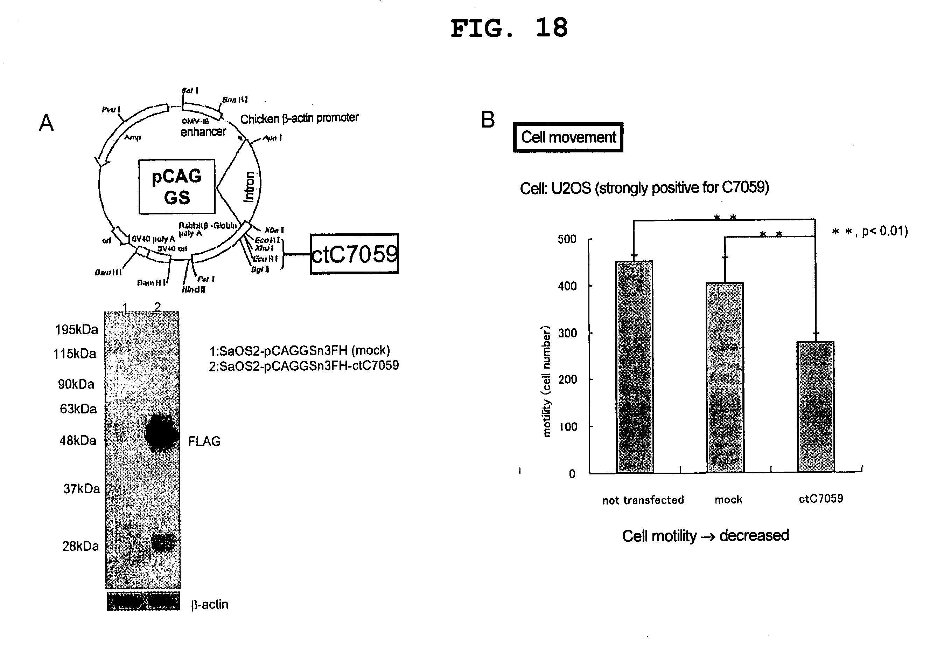

By inserting a DNA that encodes a polypeptide comprising ctC7059 and three Flag tags linked to the N-terminus thereof (3FH-ctC7059) into a multicloning site of pCAGGS, a vector capable of transiently strongly expressing the 3FH-ctC7059 protein was prepared (Fig. 18A). The 3FH-ctC7059 expression vector was introduced into a C7059 strongly positive sarcoma cell line (U2OS), and the motility of the cells was evaluated.
As a result, with introduction of the ctC7059 expression vector, the motility of the sarcoma cells decreased significantly (Fig. 18B). This result suggested that ctC7059 might possess antagonistic action on C7059 and possess an activity to inhibit the metastasis of a sarcoma.

### Example 2: Expression of sarcopodin in colorectal cancer (Methods)

The subjects were 39 patients with colic or rectal cancer who underwent surgery at Kyoto University Hospital between 1999 and the present. Separated tumor tissues were fixed with formalin, and subjected to immunohistochemical staining using the anti-C7059 antibody prepared in Example 1. A non-tumoral portion adjacent to the tumor tissue was used as a normal mucosa.

### (Results)

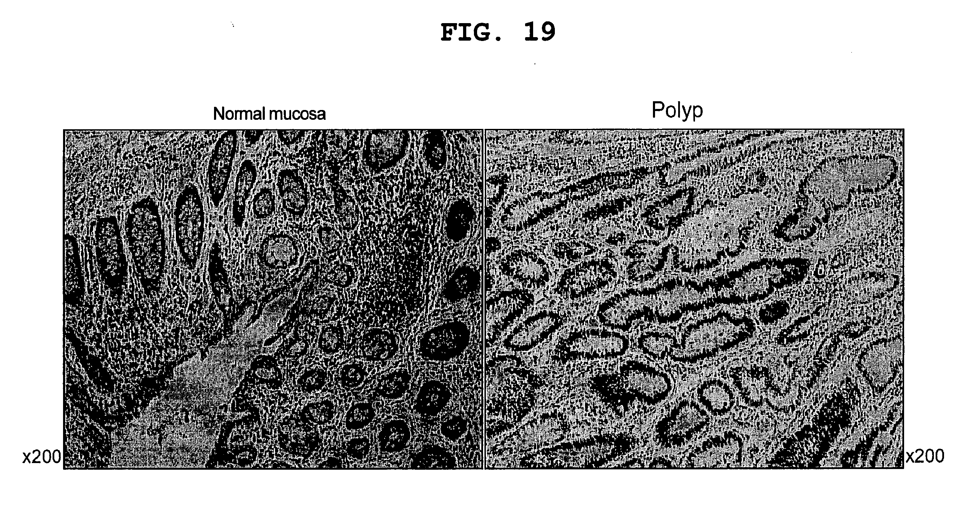

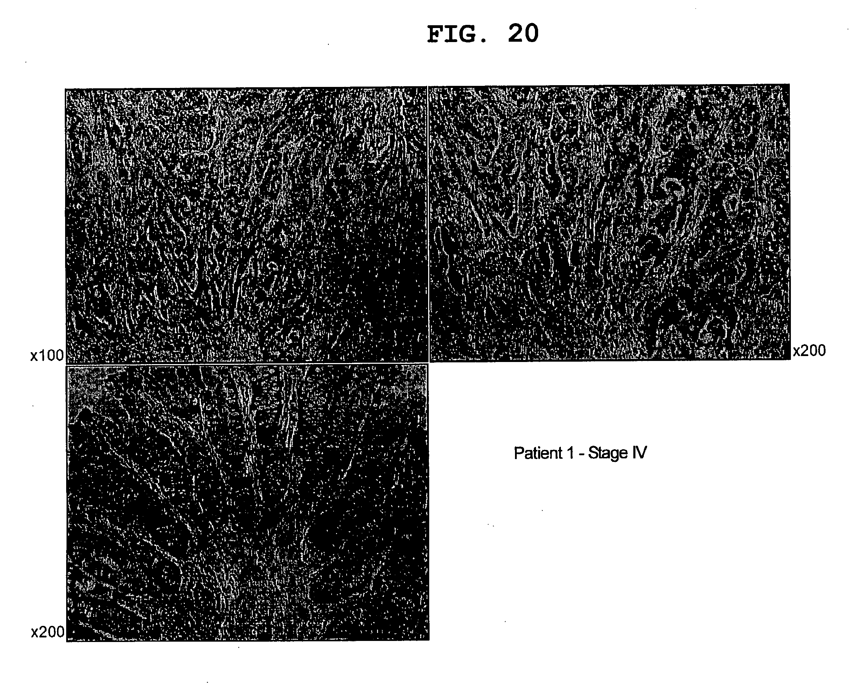

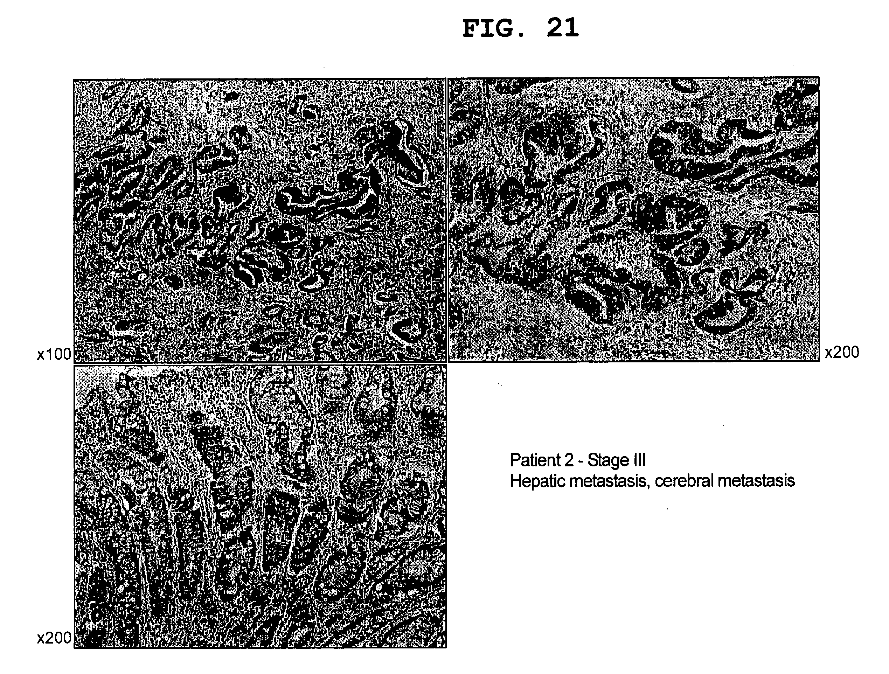

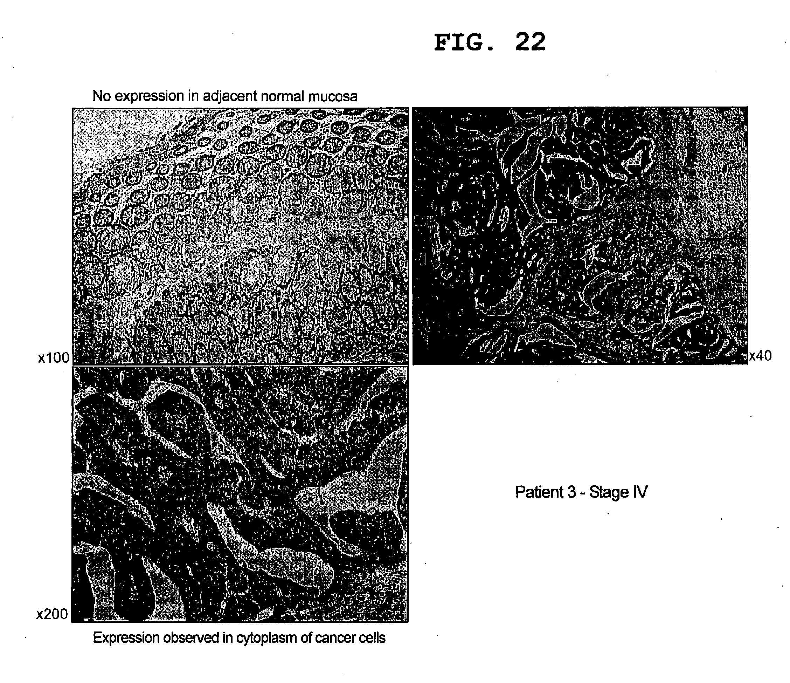

In the normal mucosa, the expression of the C7059 protein was little observed (Figs. 19 to 23). Expression localized in the nucleus was rarely observed.
In the polyps as well, the expression of the C7059 protein was not observed, or the expression of the C7059 protein localized in the nucleus was observed. No expression in the cytoplasm was observed.
In contrast, in tumor cells, the expression of the C7059 protein was observed in many patients (26/39) (Figs. 19 to 23). Although the expression in the cytoplasm was predominant, expression was also observed in the nucleus. Results of an evaluation of the stainability in the cytoplasm in four grades (no stain, weakly positive, positive, strongly positive) are as follows:
No stain: 13 (33.3%)
Weakly positive: 12 (30.1%)
Positive: 12 (30.1%)
Strongly positive: 2 (0.5%)

These results suggested that C7059 is a molecule whose expression increases with the tumorization of a colorectal mucosal cell, and that by evaluating the presence or absence of the expression of C7059, whether or not the colorectal mucosal cell is a tumor cell can be determined. It was also suggested that by evaluating whether the C7059 expression site in the cell is the cytoplasm or is localized in the nucleus, tumors and polyps in the large intestine could be distinguished from each other.

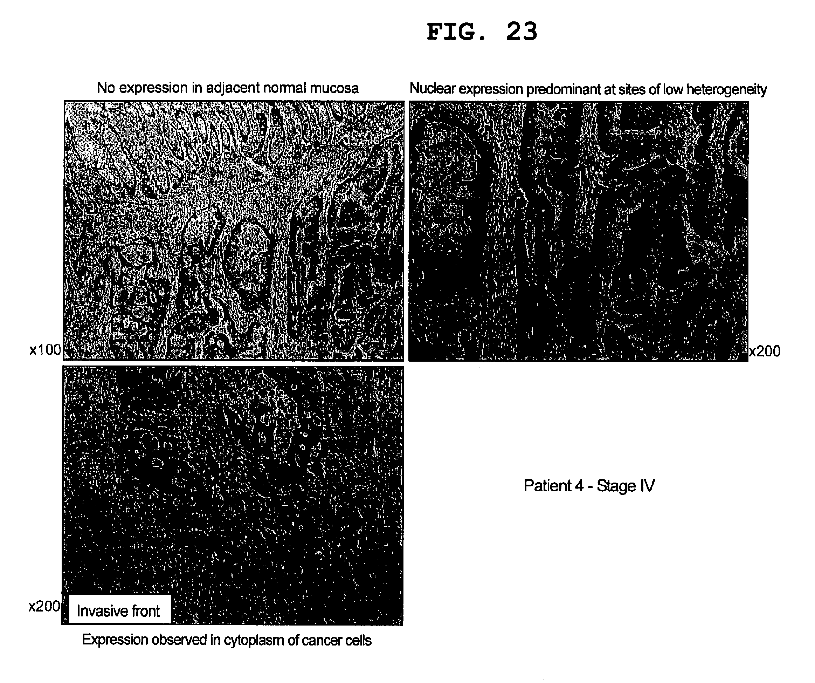

At tumor sites of low heterogeneity, C7059 expression in the nucleus was distinctive; at tumor sites of high heterogeneity, such as invasive fronts, however, C7059 expression in the cytoplasm was predominant (Fig. 23). This finding showed that in colorectal cancer, like in spindle cell sarcoma, the localization of the C7059 protein possibly varies depending on the malignancy of the cell.

Additionally, judging from these findings combined with clinical information, the positive patients were identified as patients in the poor prognosis group (Stage IV) or those experiencing a local recurrence or metastasis.
This finding suggested that by evaluating the expression level of C7059, the risk of metastasis and the prognosis for survival in colorectal cancer could be determined.

### Example 3: Expression of sarcopodin in colorectal cancer (2)

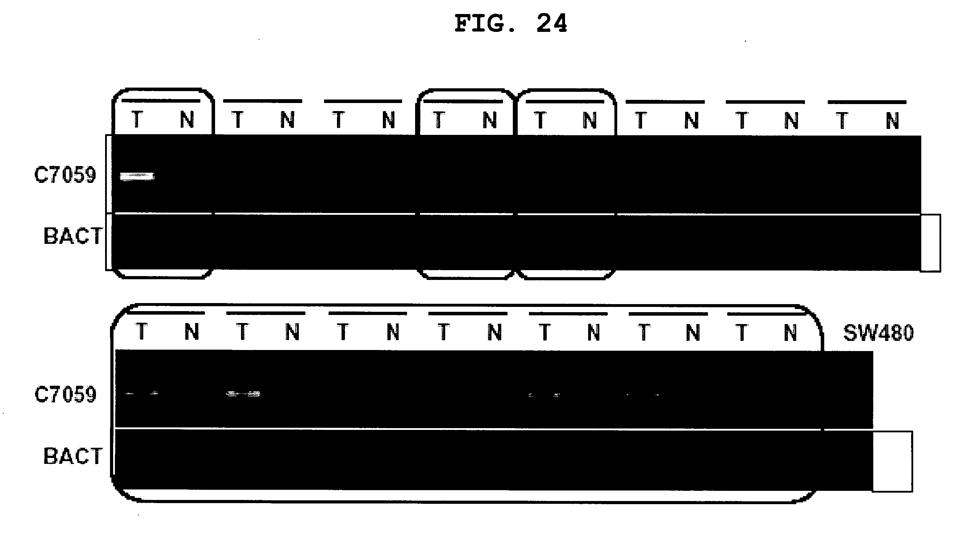

The subjects were 15 patients with colic or rectal cancer who underwent surgery at Kyoto University Hospital between 2006 and 2007. Separated normal tissue and tumor tissue of the colon-rectum were stored under freezing, and C7059 expression was measured by quantitative RT-PCR in the same manner as Example 1. As a result, in 10 of the 15 patients, C7059 expression was enhanced in the colic or rectal tumor tissue, compared with the normal colic or rectal tissue (Fig. 24).

This result suggested that C7059 is a molecule whose expression increases with the tumorization of a colorectal mucosal cell, and that by evaluating the presence or absence of the expression of C7059, whether or not the colorectal mucosal cell is a tumor cell can be determined.

### Example 4: Expression of sarcopodin in colorectal cancer (3) (Methods)

The subjects were 164 patients with colic or rectal cancer who underwent surgery at Kyoto University Hospital between 1999 and 2001. Separated tumor tissue was fixed with formalin, and subjected to immunohistochemical staining using the anti-C7059 antibody prepared in Example 1. A non-tumoral portion adjacent to the tumor tissue was used as a normal mucosa.

### (Results)

In tumor cells, in many patients, expression of the C7059 protein was observed (124/164). Results of an evaluation of the stainability in the cytoplasm in three grades (no stain, weakly positive, strongly positive) are as follows:
No stain: 40 (24.4%)
Weakly positive: 53 (32.3%)
Strongly positive: 71 (43.3%)

These results showed that C7059 is a molecule whose expression increases with the tumorization of a colorectal mucosal cell, and that by evaluating the presence or absence of the expression of C7059, whether or not the colorectal mucosal cell is a tumor cell can be determined.

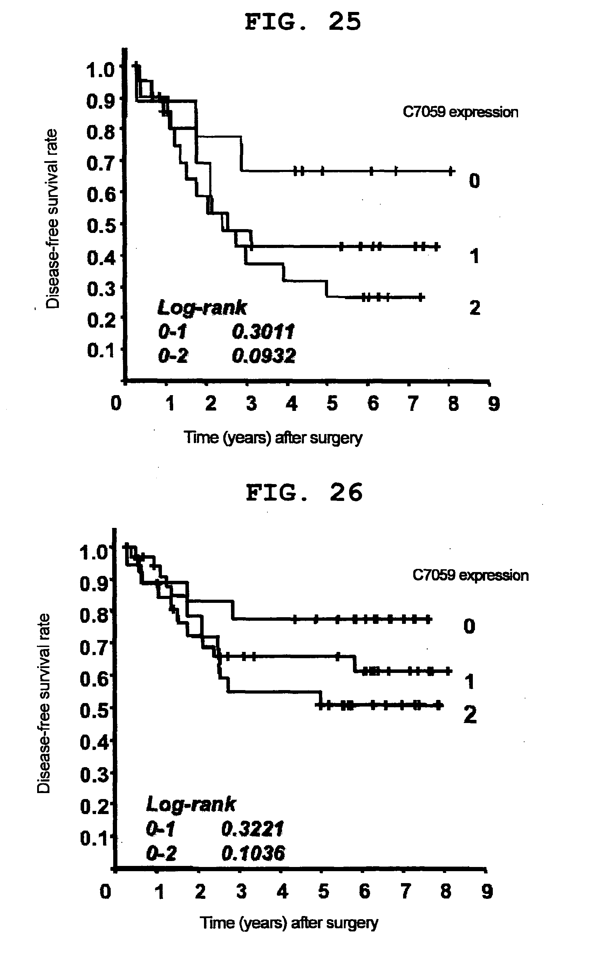

Stage III patients with strong expression of C7059 (21/52) tended to be more likely to experience a local recurrence or metastasis than patients in the same stage without expression of C7059 (9/52) (Fig. 25).

In rectal cancer patients undergoing radical surgery to resect a tumor site (80 patients), patients with high expression of C7059 tended to be more likely to experience a local recurrence or metastasis than patients without expression of C7059 (Fig. 26).

The results above showed that by evaluating the expression level of C7059, the risk of recurrence/metastasis and prognosis for survival in colorectal cancer can be determined.

### Industrial Applicability

Using the method of the present invention, it is possible to quickly determine the risk of onset of distant metastasis or prognosis for survival in a tumor such as spindle cell sarcoma or colorectal cancer using a convenient technique such as immunological staining using an antibody or quantitative RT-PCR using a specific probe. Using the method of the present invention, it is possible to administer a selective treatment to the patient on the basis of the level of the risk of onset of distant metastasis or the determination of prognosis for survival, thus improving the quality of tumor treatment. Specifically, for patients judged to have a poor prognosis using the method of the present invention, an improvement of the prognosis can be aimed at by proactively performing an adjuvant therapy, and attempting for early detection of metastatic foci by regular diagnostic imaging. Conversely, for patients judged to have a good prognosis using the method of the present invention, unnecessary adjuvant therapy can be avoided. In particular, because the method of the present invention is applicable to all types of spindle cell sarcoma, the prognosis for survival can be determined accurately even if a pathologic diagnosis is unestablished.
Using the method of the present invention, the presence or absence of colorectal cancer cells and the malignancy of colorectal cancer cells can be determined with high accuracy.
Using the agent for inhibiting metastasis of the present invention, it is possible to selectively suppress the metastasis of spindle cell sarcoma and improve the prognosis for survival in the spindle cell sarcoma. The agent for inhibiting metastasis of the present invention is distinctive in that the onset of distant metastasis, which is a factor that determines the prognosis of spindle cell sarcoma, is targeted. Because the expression levels in normal tissue are much lower than those in tumor tissue, the adverse reactions caused by expression suppressing treatment with an siRNA are expected to be very mild, and the treatment enables an effective treatment of spindle cell sarcoma when combined with currently available therapeutic methods.
This application is based on a patent application No. 2007-145827 filed in Japan (filing date: May 31, 2007), the contents of which are incorporated in full herein by this reference.

## Claims

1. A method of determining the risk of distant metastasis of a tumor, comprising measuring the expression level of any polypeptide or polynucleotide selected from among (1) to (5) below in tumor tissue, and determining the risk of distant metastasis of the tumor on the basis of the correlation between the expression level and the incidence of distant metastasis:
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell;
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(4) a polynucleotide comprising a coding region in the nucleotide sequence depicted in SEQ ID NO:1; and
(5) a polynucleotide comprising a nucleotide sequence that encodes any polypeptide selected from among (1) to (3) above.

2. The method according to claim 1, wherein the expression level is measured using any substance selected from among (i) to (iii) below:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

3. The method according to claim 2, wherein the substance used for the measurement is any one selected from among (ia) to (iiia) below:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

4. The method according to any one of claims 1 to 3, wherein the tumor is spindle cell sarcoma or colorectal cancer.

5. A method of determining the prognosis for survival of a tumor patient, comprising measuring the expression level of any polypeptide or polynucleotide selected from among (1) to (5) below in tumor tissue, and determining the prognosis for survival of the tumor patient on the basis of the correlation between the expression level and the determination of the prognosis for survival of the tumor patient:
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell;
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(4) a polynucleotide comprising a coding region in the nucleotide sequence depicted in SEQ ID NO:1; and
(5) a polynucleotide comprising a nucleotide sequence that encodes any polypeptide selected from among (1) to (3) above.

6. The method according to claim 5, wherein the expression level is measured using any substance selected from among (i) to (iii) below:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

7. The method according to claim 6, wherein the substance used for the measurement is any one selected from among (ia) to (iiia) below:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

8. The method according to any one of claims 5 to 7, wherein the tumor is spindle cell sarcoma or colorectal cancer.

9. An agent for determining the risk of distant metastasis of a tumor, comprising any substance selected from among (i) to (iii) below:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

10. The agent according to claim 9, wherein the substance is any one selected from among (ia) to (iiia) below:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

11. The agent according to claim 9 or 10, wherein the tumor is spindle cell sarcoma or colorectal cancer.

12. An agent for determining the prognosis for survival of a tumor patient, comprising any substance selected from among (i) to (iii) below:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

13. The agent according to claim 12, wherein the substance is any one selected from among (ia) to (iiia) below:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

14. The agent according to claim 12 or 13, wherein the tumor is spindle cell sarcoma or colorectal cancer.

15. A method of detecting colorectal cancer cells in colorectal mucosal tissue, comprising evaluating the expression of any polypeptide or polynucleotide selected from among (1) to (5) below in colorectal mucosal tissue, and determining whether or not colorectal cancer cells are contained in the tissue on the basis of the presence or absence of the expression:
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell;
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(4) a polynucleotide comprising a coding region in the nucleotide sequence depicted in SEQ ID NO:1; and
(5) a polynucleotide comprising a nucleotide sequence that encodes any polypeptide selected from among (1) to (3) above.

16. An agent for detecting colorectal cancer cells in colorectal mucosal tissue, comprising any substance selected from among (i) to (iii) below:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

17. A method of determining the malignancy of a tumor cell, comprising evaluating the localization of any polypeptide selected from among (1) to (3) below in the tumor cell, and determining the malignancy of the tumor cell on the basis of the presence or absence of the expression of the polypeptide in the cytoplasm:
(1) a polypeptide comprising the amino acid sequence depicted in SEQ ID NO:2;
(2) a polypeptide comprising an amino acid sequence having an identity of 90% or more to the amino acid sequence depicted in SEQ ID NO:2, and possessing an activity to enhance the motility or invasive potential of a cell when expressed in the cell; and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell.

18. The method according to claim 17, wherein the tumor is spindle cell sarcoma or colorectal cancer.

19. An agent for determining the malignancy of a tumor cell, comprising an antibody that specifically recognizes a polypeptide consisting of the amino acid sequence depicted in SEQ ID NO:2.

20. The agent according to claim 19, wherein the tumor is spindle cell sarcoma or colorectal cancer.

21. An agent for inhibiting sarcoma metastasis comprising any polynucleotide selected from among (a) to (d) below or an expression vector capable of expressing the polynucleotide:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell, and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

22. An agent for inhibiting the motility or invasive potential of a sarcoma cell, comprising any polynucleotide selected from among (a) to (d) below or an expression vector capable of expressing the polynucleotide:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell, and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide comprising the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

23. A method of screening for a substance capable of inhibiting the metastasis of a sarcoma or the motility or invasive potential of a sarcoma cell, comprising the following steps of:
(I) bringing into contact with each other a test substance and a cell that permits a measurement of the expression of any polypeptide or polynucleotide selected from among (1) to (5) below:
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell,
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell,
(4) a polynucleotide comprising a coding region in the nucleotide sequence depicted in SEQ ID NO:1, and
(5) a polynucleotide comprising a nucleotide sequence that encodes any polypeptide selected from among (1) to (3) above;
(II) measuring the amount of any polypeptide or polynucleotide selected from among (1) to (5) above expressed in the cell contacted with the test substance, and comparing this amount expressed with the amount of the above-described polypeptide or polynucleotide expressed in a control cell not contacted with the test substance; and
(III) selecting a test substance that suppresses the amount of any polypeptide or polynucleotide selected from among (1) to (5) above expressed, as a substance capable of inhibiting the metastasis of a sarcoma or the motility or invasive potential of a sarcoma cell, on the basis of the results of the comparison in (II) above.

24. An agent for inhibiting the metastasis of a sarcoma, comprising any polypeptide selected from among (1) to (3) below or an expression vector capable of expressing the polypeptide:
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.

25. An agent for inhibiting the motility of a sarcoma cell, comprising any polypeptide selected from among (1) to (3) below or an expression vector capable of expressing the polypeptide:
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.

26. A method of screening for a peptide capable of inhibiting the metastasis of a sarcoma or the motility of a sarcoma cell, comprising the following steps of:
(I) expressing a test polypeptide comprising at least three consecutive amino acids contained in the amino acid sequence depicted in SEQ ID NO:2 in a sarcoma cell expressing a polypeptide comprising the amino acid sequence depicted in SEQ ID NO:2;
(II) measuring the motility of the cell in which the test polypeptide has been expressed, and comparing this motility with the motility of a control sarcoma cell in which the test polypeptide has not been expressed;
(III) selecting a polypeptide that has suppressed the motility of the sarcoma cell as a polypeptide capable of inhibiting the metastasis of a sarcoma or the motility of a sarcoma cell, on the basis of the results of the comparison in (II) above.

27. Any substance selected from among (i) to (iii) below for determining the risk of distant metastasis of a tumor:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

28. The substance according to claim 27, which is any one selected from among (ia) to (iiia) below:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

29. The substance according to claim 27 or 28, wherein the tumor is spindle cell sarcoma or colorectal cancer.

30. Any substance selected from among (i) to (iii) below for determining the prognosis for survival of a tumor patient:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence shown by SEQ ID NO:2.

31. The substance according to claim 30, wherein the substance is any one selected from among (ia) to (iiia) below:
(ia) an antibody that specifically recognizes a polypeptide consisting of the amino acid sequence depicted in SEQ ID NO:2,
a recognition epitope for the antibody being present within the region shown by amino acid numbers 1 to 385 in SEQ ID NO:2;
(iia) a nucleic acid probe or primer capable of specifically detecting a polynucleotide consisting of the nucleotide sequence shown by nucleotide numbers 1 to 1229 in SEQ ID NO:1; and
(iiia) a nucleic acid probe or primer capable of specifically detecting a polynucleotide consisting of a nucleotide sequence that encodes a polypeptide consisting of the amino acid sequence shown by amino acid numbers 1 to 385 in SEQ ID NO:2.

32. The substance according to claim 30 or 31, wherein the tumor is spindle cell sarcoma or colorectal cancer.

33. Any substance selected from among (i) to (iii) below, for detecting colorectal cancer cells in colorectal mucosal tissue:
(i) an antibody that specifically recognizes a polypeptide consisting of the amino acid sequence depicted in SEQ ID NO:2;
(ii) a nucleic acid probe or primer capable of specifically detecting a polynucleotide consisting of the nucleotide sequence depicted in SEQ ID NO:1; and
(iii) a nucleic acid probe or primer capable of specifically detecting a polynucleotide consisting of a nucleotide sequence that encodes a polypeptide consisting of the amino acid sequence depicted in SEQ ID NO:2.

34. An antibody that specifically recognizes a polypeptide consisting of the amino acid sequence depicted in SEQ ID NO:2, for determining the malignancy of a tumor cell.

35. The antibody according to claim 34, wherein the tumor is spindle cell sarcoma or colorectal cancer.

36. Any polynucleotide selected from among (a) to (d) below or an expression vector capable of expressing the polynucleotide, for inhibiting the metastasis of a sarcoma:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell, and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

37. Any polynucleotide selected from among (a) to (d) below or an expression vector capable of expressing the polynucleotide, for inhibiting the motility or invasive potential of a sarcoma cell:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell, and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

38. Any polypeptide selected from among (1) to (3) below or an expression vector capable of expressing the polypeptide, for inhibiting the metastasis of a sarcoma:
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.

39. Any polypeptide selected from among (1) to (3) below or an expression vector capable of expressing the polypeptide, for inhibiting the motility of a sarcoma cell:
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.

40. A method for inhibiting the metastasis of a sarcoma in a mammal, comprising administering to the mammal an effective amount of any polynucleotide selected from among (a) to (d) below or an expression vector capable of expressing the polynucleotide:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell, and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

41. A method for inhibiting the motility or invasive potential of a sarcoma cell in a mammal, comprising administering to the mammal an effective amount of any polynucleotide selected from among (a) to (d) below or an expression vector capable of expressing the polynucleotide:
(a) a polynucleotide comprising a nucleotide sequence complementary to the nucleotide sequence depicted in SEQ ID NO:1, the nucleotide sequence of an initial transcription product that produces the former nucleotide sequence by post-transcriptional processing, or a partial sequence thereof as long as 15 bases or more;
(b) a polynucleotide comprising a nucleotide sequence complementary to a nucleotide sequence that encodes any polypeptide selected from among (1) to (3) below, the nucleotide sequence of an initial transcription product that produces the former nucleotide sequence by post-transcriptional processing, or a partial sequence thereof as long as 15 bases or more;
(1) a polypeptide comprising the amino acid sequence depicted in SEQ ID NO:2,
(2) a polypeptide comprising an amino acid sequence having an identity of 90% or more to the amino acid sequence depicted in SEQ ID NO:2, and possessing an activity to enhance the motility or invasive potential of a cell when expressed in the cell, and
(3) a polypeptide comprising an amino acid sequence resulting from deletion, substitution, insertion or addition of one or a plurality of amino acids in the amino acid sequence depicted in SEQ ID NO:2, and possessing an activity to enhance the motility or invasive potential of a cell when expressed in the cell;
(c) a polynucleotide comprising a nucleotide sequence hybridizable, under physiological conditions for an animal which is the subject of treatment, with a polynucleotide consisting of the nucleotide sequence depicted in SEQ ID NO:1, or an initial transcription product that produces the former polynucleotide by post-transcriptional processing, and suppressing the transcription or translation of a polypeptide consisting of the amino acid sequence depicted in SEQ ID NO:2 in the hybridized state; and
(d) a polynucleotide comprising a nucleotide sequence hybridizable, under physiological conditions for an animal which is the subject of treatment, with a polynucleotide consisting of a nucleotide sequence that encodes any polypeptide selected from among (1) to (3) above, or an initial transcription product that produces the former nucleotide sequence by post-transcriptional processing, and suppressing the transcription or translation of any one of the polypeptides (1) to (3) in the hybridized state.

42. A method for inhibiting the metastasis of a sarcoma in a mammal, comprising administering to the mammal an effective amount of any polypeptide selected from among (1) to (3) below or an expression vector capable of expressing the polypeptide:
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.

43. A method for inhibiting the motility of a sarcoma cell in a mammal, comprising administering to the mammal an effective amount of any polypeptide selected from among (1) to (3) below or an expression vector capable of expressing the polypeptide:
(1) a polypeptide comprising the amino acid sequence depicted in SEQ ID NO:4;
(2) a polypeptide comprising an amino acid sequence having an identity of 90% or more to the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell; and
(3) a polypeptide comprising an amino acid sequence resulting from deletion, substitution, insertion or addition of one or a plurality of amino acids in the amino acid sequence depicted in SEQ ID NO:4, and possessing an activity to inhibit the motility of a sarcoma cell expressing a polypeptide comprising the amino acid sequence depicted in SEQ ID NO:2 when expressed in the sarcoma cell.
